# EUROPEAN PATENT SPECIFICATION

(11) **EP 1 943 969 B1**
(45) Date of publication and mention of the grant of the patent: **02.09.2009**
(21) Application number: 08000654.7
(22) Date of filing: 15.01.2008
(51) Int. Cl.: A61B 17/32, A61B 18/14

(54) **Ultrasonic operating apparatus**
Ultraschalloperationsvorrichtung
Appareil à fonctionnement à ultrasons

(30) Priority: 15.01.2007 US 623228
(43) Date of publication of application: 16.07.2008
(73) Proprietor: Olympus Medical Systems Corp., Tokyo 151-0072 (JP)
(72) Inventor: Masuda, Shinya, Hachioji-shi Tokyo 192-8512 (JP); Yachi, Chie, Hachioji-shi Tokyo 192-8512 (JP); Taniguchi, Kazunori, Hachioji-shi Tokyo 192-8512 (JP)
(74) Representative: von Hellfeld, Axel

(56) References cited:
- EP-A- 1 327 420
- US-A1- 2005 234 338
- US-A1- 2006 241 532

## Description

The present invention relates to an ultrasonic operating apparatus capable of administering a treatment such as incision, removal or coagulation of a living tissue by use of ultrasonic waves and also capable of administering a treatment with a high frequency.

An ultrasonic operating apparatus described in, for example, Jpn. Pat. Appln. KOKAI Publication No. 2003-265496 (Patent document 1) and EP 1 327 420 A1 has been disclosed as one example of an ultrasonic operating apparatus generally capable of administering a treatment such as incision, removal or coagulation of a living tissue by use of ultrasonic waves and also capable of administering a treatment with a high frequency.

In this apparatus, an operation portion at hand is coupled to the proximal end of an elongate insertion portion. An ultrasonic transducer for generating ultrasonic vibrations is provided in this operation portion. A treatment portion for treating the living tissue is provided at the distal end of the insertion portion.

The insertion portion has an elongate circular tubular sheath. A vibration transmitting member is inserted in the sheath. The proximal end of the vibration transmitting member is detachably connected to the ultrasonic transducer via a threaded joint. Thus, the ultrasonic vibrations generated by the ultrasonic transducer are transmitted to an ultrasonic probe on the distal side of the vibration transmitting member.

In the treatment portion, a jaw is provided opposite to the ultrasonic probe. The proximal end of the jaw is swingably supported on the distal end of the sheath via a support shaft. An operation rod for driving the jaw is inserted in the sheath to be able to axially move back and forth. An operation handle is provided in the operation portion. Thus, the operation rod is driven to axially move back and forth by the operation of the operation handle, and the jaw is opened/closed with respect to the ultrasonic probe in conjunction with the operation of this operation rod.

At this point, the living tissue is gripped between the ultrasonic probe and the jaw in accordance with the closing of the jaw. In this state, the ultrasonic vibrations from the ultrasonic transducer is transmitted to the ultrasonic probe on the side of the treatment portion via the vibration transmitting member, thereby administering the treatment such as incision, removal or coagulation of the living tissue by use of the ultrasonic waves.

Furthermore, in the apparatus of Patent document 1 described above, the proximal end of the sheath is detachably coupled to the operation handle of the operation portion. Moreover, a high-frequency connecting pin is attached to the operation portion. An electric cord for supplying a high-frequency current from a high-frequency cauterization power supply unit is connected to the high-frequency connecting pin. The inner end of the high-frequency connecting pin is electrically connected to the ultrasonic probe of the treatment portion via the operation portion and via an electric conduction path within the sheath, or to the jaw. Thus, the high-frequency current is supplied to the ultrasonic probe of the treatment portion or to the jaw as necessary, so that a high-frequency treatment such as the coagulation of the living tissue is administered.

The apparatus of Patent document 1 described above uses a type called a monopolar type in which return electrodes are disposed on the outside of the body of a patient when the high-frequency treatment is administered. Thus, the high-frequency current is passed from a treatment tool to the return electrodes through the living tissue when the high-frequency treatment is administered.

Furthermore, an apparatus which has a configuration incorporating a high-frequency treatment tool of a type called a bipolar type has also been developed as another example of the ultrasonic operating apparatus capable of administering a treatment with a high frequency. In the configuration of this type of treatment tool, a pair of electrically insulated electrodes is provided in a treatment portion at the distal end of an insertion portion. Thus, a high-frequency current is passed across the two electrodes while the pair of electrodes is in contact with the living tissue at the same time, thereby achieving high-frequency heating of the living tissue.

Where a high-frequency treatment device referred to as a bipolar type is incorporated in an ultrasonic operating apparatus from which a sheath and a handle are detachable as disclosed in Patent document 1, the conduction of a high-frequency current in an electric conduction path is inevitably unstable at the portions where the sheath and the handle are attached or detached.

In the structure of an ultrasonic operating apparatus incorporating a bipolar type high-frequency treatment device, the cables for the ultrasonic elements and the cables for the bipolar elements are prepared independently of each other. For this reason, the cables for the bipolar elements are connected to the hand piece of the ultrasonic operating apparatus, in addition to the cables for the ultrasonic elements. Since the number of cables connected to the hand piece is thus increased, the hand piece may not be easily operated.

The present invention has been made in consideration of the above circumstances, and an object of the present invention is to provide an ultrasonic operating apparatus which prevents the conduction of a high-frequency current in an electric conduction path from becoming unstable at the portions where the sheath and the handle are attached or detached, and which enables reduction of the number of cables to be connected to the hand piece, thereby ensuring an easy operation of the hand piece.

The invention is defined in appended claim 1. Preferred embodiments are described in the dependent claims.

Preferably, the outer peripheral flange portion has an outer peripheral portion formed into a non-circular irregular shape; and the urging portion is disposed inside the handle portion, the urging portion being held at the non-pressure-contact position with respect to the outer peripheral flange portion at an insertion operation position at which the outer peripheral flange portion is inserted into the insertion hole forming portion along the axial direction of the sheath portion, the urging portion being switched to the pressure-contact position along with the rotational operation of the outer peripheral flange portion from the insertion operation position in a direction around the central axis of the sheath portion.

Preferably, the urging portion has a ring-shaped rubber portion; and the rubber portion is brought into pressure-contact with the outer peripheral flange portion to urge the outer peripheral flange portion in a direction to permit conduction between the sheath portion side electric path and the handle portion side electric path when the sheath portion is connected to the handle portion.

Preferably, the rubber portion is formed by conductive rubber.

Preferably, the urging portion has a leaf-spring-shaped urging member; and the urging member is brought into pressure-contact with the outer peripheral flange portion to urge the outer peripheral flange portion in a direction to permit conduction between the sheath portion side electric path and the handle portion side electric path when the sheath portion is connected to the handle portion.

An ultrasonic operating apparatus in another aspect of the present invention comprises: an ultrasonic transducer which generates ultrasonic vibrations; a probe portion which has a distal end and a proximal end, the proximal end being coupled to the ultrasonic transducer, ultrasonic waves output from the ultrasonic transducer being transmitted to the probe portion; a first high-frequency electric path which is provided in a combination of the ultrasonic transducer and the probe portion and which transmits a high-frequency current; a sheath portion which is formed by a cylindrical member having a distal end and a proximal end and into which the probe portion is removably inserted, the sheath portion having a jaw swingably supported on the distal end thereof to be opposite to the probe portion; a handle portion which is detachably coupled to the proximal end of the sheath portion and which opens/closes the jaw with respect to the probe portion, the handle portion having a transducer connecting portion to which the ultrasonic transducer is detachably connected; and a second high-frequency electric path which is provided in a combination of the sheath portion and the handle portion and which transmits a high-frequency current, the second high-frequency electric path having: a sheath portion side electric path disposed on the side of the sheath portion; a handle portion side electric path disposed on the side of the handle portion; and an electric connection portion which performs the operation of connecting the sheath portion side electric path and the handle portion side electric path along with the operation of connecting the sheath portion and the handle portion, the electric connection portion having: an outer peripheral flange portion which is disposed at the proximal end of the sheath portion and which is formed on the outer periphery of the sheath portion and which is connected to the sheath portion side electric path; and an engaging portion which is disposed inside the handle portion and which removably engages with the outer peripheral flange portion, the engaging portion being equipped with: an insertion hole forming portion into which the outer peripheral flange portion is inserted when the sheath portion is coupled to the handle portion; and an urging portion disposed in the insertion hole forming portion, the urging portion being switched between a non-pressure-contact position where the urging portion is held out of pressure-contact with the outer peripheral flange portion along with the operation of relatively rotating the handle portion and the sheath portion in a direction around the central axis of the sheath portion and a pressure-contact position where the urging portion is brought into pressure-contact with the outer peripheral flange portion, the urging portion permitting conduction between the sheath portion side electric path and the handle portion side electric path at the pressure-contact position.

Preferably, the handle portion has a rotational operation portion which rotationally drives the sheath portion in a direction around the central line of the sheath portion; and the transducer connecting portion has a tubular member formed by an insulating material which permits insulation between the probe portion and the second high-frequency electric path when the probe portion is connected to the handle portion.

Preferably, the tubular member has a position regulating mechanism which regulates the rotational position of the probe when the probe portion is connected to the handle portion.

Preferably, the position regulating mechanism has a protrusion provided to inwardly protrude on the inner peripheral surface of the tubular member; and an engaging concave portion which is formed in the outer peripheral surface of the probe portion and which engages with the protrusion of the tubular member.

Preferably, the probe portion has, at the distal end thereof, a distal end curved portion which is curved in a direction deviating from the direction of the central line of the probe portion; and the jaw has a distal end curved portion which is curved to have a shape corresponding to the distal end curved portion of the probe portion.

Preferably, the handle portion has a rotational operation portion which rotationally drives the sheath portion in a direction around the central line of the sheath portion; and the sheath portion has a tubular member formed by an insulating material which permits insulation between the probe portion and the second high-frequency electric path when the probe portion is connected to the handle portion.

The invention can be more fully understood from the following detailed description when taken in conjunction with the accompanying drawings, in which:
FIG. 1 is a perspective view showing an overall schematic configuration of an ultrasonic operating apparatus in a first embodiment of the present invention;
FIG. 2 is a perspective view showing a situation where coupling parts of the ultrasonic operating apparatus in the first embodiment are detached;
FIG. 3A is a plan view showing the distal end of a sheath unit of the ultrasonic operating apparatus in the first embodiment;
FIG. 3B is a plan view showing the distal end of a probe unit of the ultrasonic operating apparatus in the first embodiment;
FIG. 4A is a longitudinal sectional view showing the distal end of the sheath unit of the ultrasonic operating apparatus in the first embodiment;
FIG. 4B is a longitudinal sectional view showing an insulating coating on the inner peripheral surface of an inner cylinder;
FIG. 5 is a sectional view along the V-V line in FIG. 4A;
FIG. 6 is a sectional view along the VI-VI line in FIG. 4A;
FIG. 7 is a sectional view along the VII-VII line in FIG. 4A;
FIG. 8 is a longitudinal sectional view showing the proximal end of the sheath unit of the ultrasonic operating apparatus in the first embodiment;
FIG. 9A is a sectional view along the IXA-IXA line in FIG. 8;
FIG. 9B is a sectional view along the IXB-IXB line in FIG. 8;
FIG. 10 is a sectional view along the X-X line in FIG. 8;
FIG. 11 is a sectional view along the XI-XI line in FIG. 8;
FIG. 12 is a perspective view showing a connecting pipe member of the sheath unit of the ultrasonic operating apparatus in the first embodiment;
FIG. 13 is a side view showing the connecting pipe member of the sheath unit of the ultrasonic operating apparatus in the first embodiment;
FIG. 14 is a side view showing how a handle unit and a transducer unit of the ultrasonic operating apparatus in the first embodiment are coupled together;
FIG. 15 is a longitudinal sectional view showing a unit coupling part of the ultrasonic operating apparatus in the first embodiment;
FIG. 16 is a longitudinal sectional view showing an internal configuration of the handle unit of the ultrasonic operating apparatus in the first embodiment;
FIG. 17A is a sectional view along the 17-17 line in FIG. 16 showing a state before engagement between the handle unit and the sheath unit of the ultrasonic operating apparatus in the first embodiment;
FIG. 17B is a sectional view along the 17-17 line in FIG. 16 showing a state after engagement between the handle unit and the sheath unit of the ultrasonic operating apparatus in the first embodiment;
FIG. 18 is a sectional view along the 18-18 line in FIG. 16;
FIG. 19 is a sectional view along the 19-19 line in FIG. 16;
FIG. 20 is a sectional view along the 20-20 line in FIG. 16;
FIG. 21 is a sectional view along the 21-21 line in FIG. 16;
FIG. 22 is a sectional view along the 22-22 line in FIG. 16;
FIG. 23 is a sectional view along the 23-23 line in FIG. 16;
FIG. 24 is a sectional view along the 24-24 line in FIG. 16;
FIG. 25 is a sectional view along the 25-25 line in FIG. 16;
FIG. 26 is a perspective view showing an electrode holding member of the ultrasonic operating apparatus in the first embodiment;
FIG. 27 is a front view showing the electrode holding member of the ultrasonic operating apparatus in the first embodiment;
FIG. 28 is a side view showing the electrode holding member of the ultrasonic operating apparatus in the first embodiment;
FIG. 29 is a perspective view showing an electrode member of the ultrasonic operating apparatus in the first embodiment;
FIG. 30 is a horizontal sectional view showing the electrode member of the ultrasonic operating apparatus in the first embodiment;
FIG. 31 is a perspective view showing a state before rotational engagement when the handle unit and the sheath unit of the ultrasonic operating apparatus in the first embodiment are coupled together;
FIG. 32 is a plan view showing a state before rotational engagement when the handle unit and the sheath unit of the ultrasonic operating apparatus in the first embodiment are coupled together;
FIG. 33 is a perspective view showing a state after rotational engagement when the handle unit and the sheath unit of the ultrasonic operating apparatus in the first embodiment are coupled together;
FIG. 34 is a plan view showing a state after rotational engagement when the handle unit and the sheath unit of the ultrasonic operating apparatus in the first embodiment are coupled together;
FIG. 35 is a side view showing a state before a set member is set to a base member of a fixed handle of the handle unit of the ultrasonic operating apparatus in the first embodiment;
FIG. 36 is a plan view showing the probe unit of the ultrasonic operating apparatus in the first embodiment;
FIG. 37 is a sectional view along the 37-37 line in FIG. 36;
FIG. 38 is a plan view showing how the transducer unit and a cable of the ultrasonic operating apparatus in the first embodiment are coupled together;
FIG. 39 is a plan view showing the proximal end of the transducer unit cable of the ultrasonic operating apparatus in the first embodiment;
FIG. 40 is a schematic configuration diagram showing electric paths of the transducer unit of the ultrasonic operating apparatus in the first embodiment;
FIG. 41 is a longitudinal sectional view showing an internal configuration of the front end of the transducer unit of the ultrasonic operating apparatus in the first embodiment;
FIG. 42A is a horizontal sectional view showing a state before the deformation of leaf spring members according to a first modification in an attachment/detachment portion between the handle unit and the sheath unit of the ultrasonic operating apparatus in the first embodiment;
FIG. 42B is a horizontal sectional view showing a state after the deformation of the leaf spring members in the same modification;
FIG. 43 is a horizontal sectional view showing an urging member of a second modification in the attachment/detachment portion between the handle unit and the sheath unit of the ultrasonic operating apparatus in the first embodiment;
FIG. 44 is a side view showing how a handle unit and a transducer unit of an ultrasonic operating apparatus in a second embodiment of the present invention are coupled together;
FIG. 45 is a longitudinal sectional view showing an internal configuration of the handle unit of the ultrasonic operating apparatus in the second embodiment; and
FIG. 46 is a longitudinal sectional view showing a connection state of internal electric paths of the handle unit of the ultrasonic operating apparatus in the second embodiment.

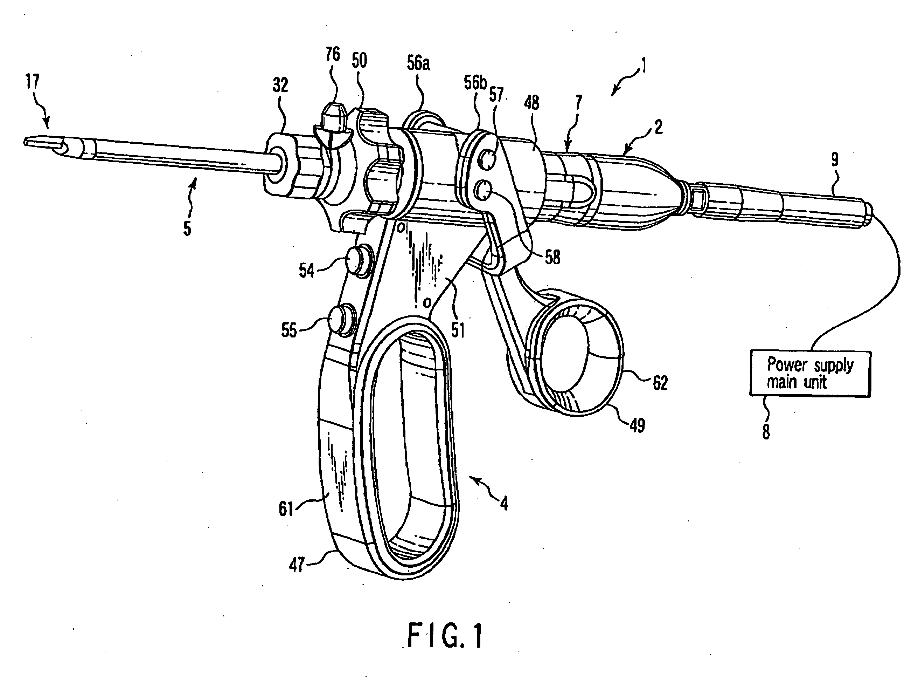

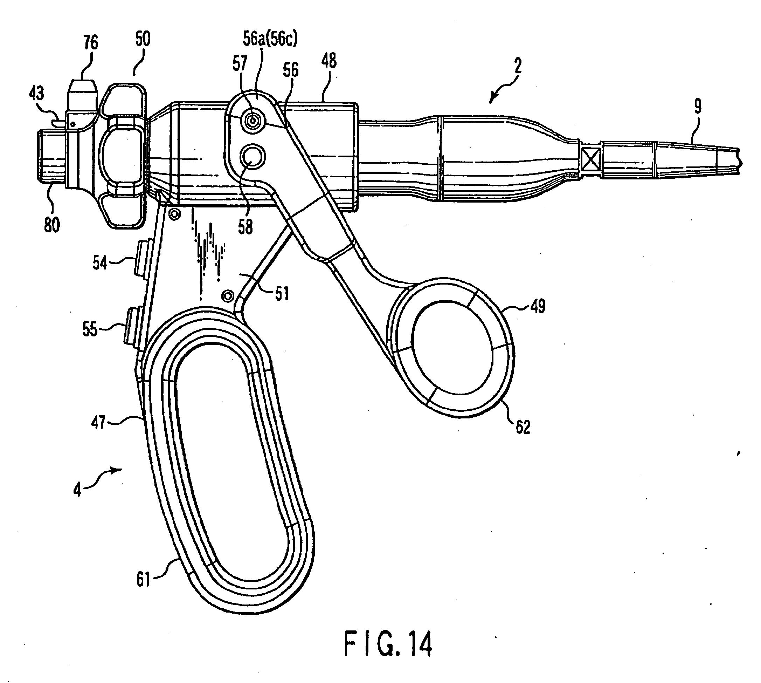

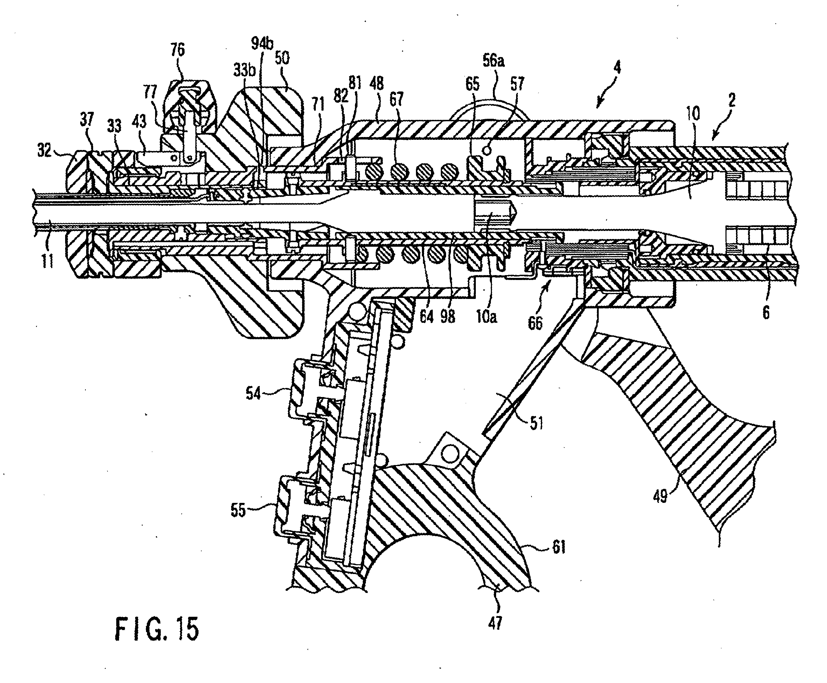

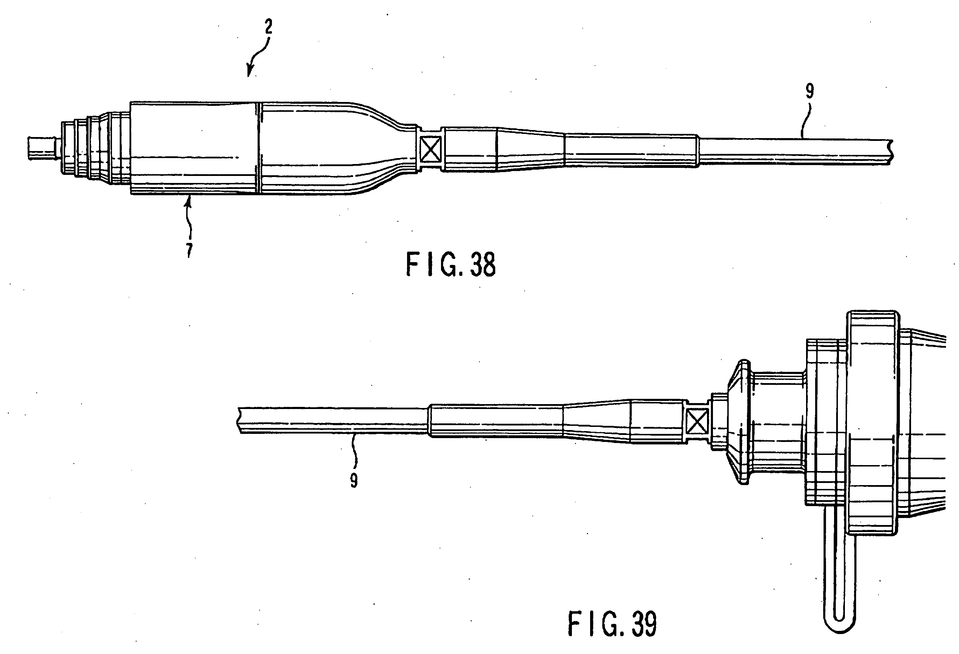

Hereinafter, a first embodiment of the present invention will be described with reference to FIG. 1 to FIG. 41. FIG. 1 shows an overall schematic configuration of a hand piece 1 of an ultrasonic operating apparatus in the present embodiment. The ultrasonic operating apparatus in the present embodiment is an ultrasonic coagulation/incision operating apparatus capable of administering a treatment such as incision, removal or coagulation of a living tissue by use of ultrasonic waves and also capable of administering a treatment with a high frequency.

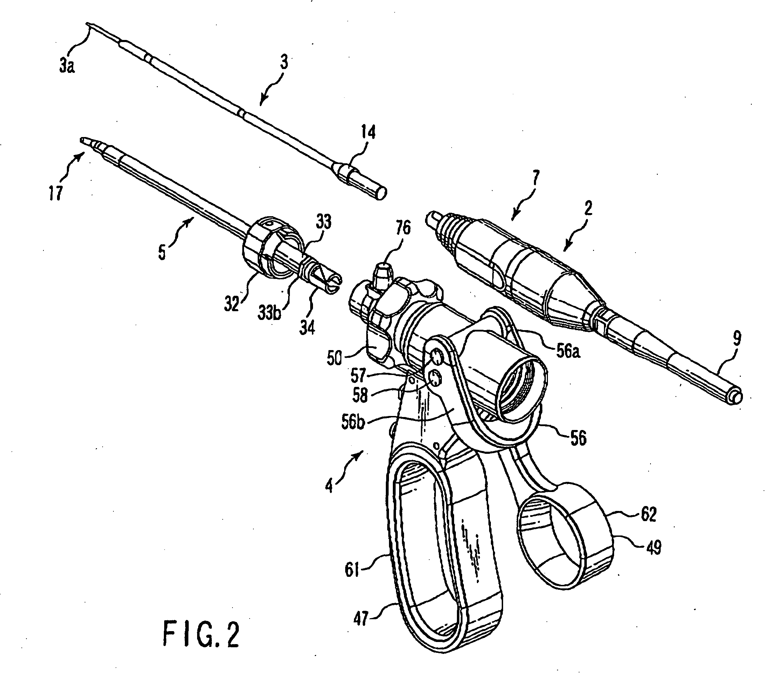

As shown in FIG. 2, the hand piece 1 has four units: a transducer unit 2, a probe unit (probe portion) 3, a handle unit (handle portion) 4, and a sheath unit (sheath portion) 5. These four units are removably coupled to each other.

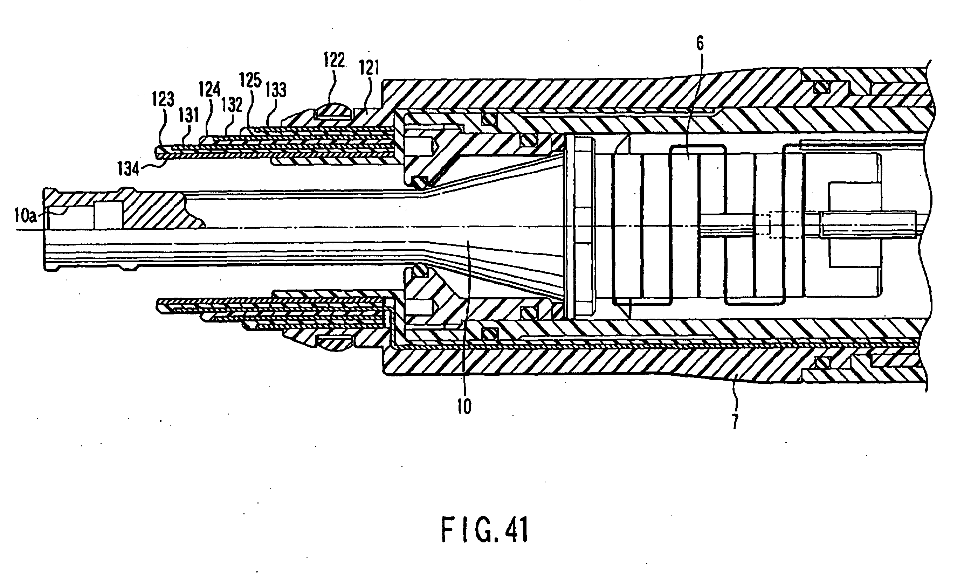

In the transducer unit 2, there is incorporated a transducer 6 (see FIG. 41) described later for generating ultrasonic vibrations by a piezoelectric element which converts an electric current into the ultrasonic vibrations. The outside of the piezoelectric element is covered with a cylindrical transducer cover 7. Further, at the rear end of the transducer unit 2, a cable 9 extends to supply from a power supply main unit 8 an electric current for generating the ultrasonic vibrations.

The proximal end of a horn 10 for amplifying/expanding the ultrasonic vibrations is coupled to the front end of the ultrasonic transducer 6 within the transducer cover 7. A screw hole 10a for attaching a probe is formed at the distal end of the horn 10.

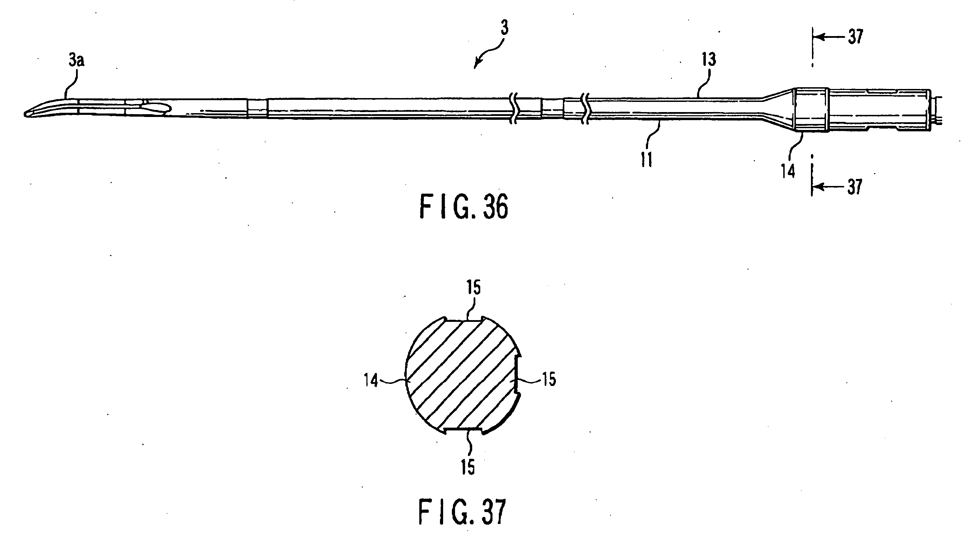

FIG. 36 shows an overall external appearance of the probe unit 3. This probe unit 3 is designed so that its entire length may be the integral multiple of the half-wave length of the ultrasonic vibrations. The probe unit 3 has a rod-like vibration transmitting member 11 made of a metal. A screw portion 12 for screwing into the screw hole 10a of the horn 10 is provided at the proximal end of the vibration transmitting member 11. Further, this screw portion 12 is threadably attached to the screw hole 10a of the horn 10 in the transducer unit 2. This sets the probe unit 3 and the transducer unit 2 together. At this point, a first high-frequency electric path 13 for transmitting a high-frequency current is formed in a combination of the ultrasonic transducer 6 and the probe unit 3.

A probe distal end 3a is provided at the distal end of the vibration transmitting member 11. The probe distal end 3a is formed to have a substantially J-shaped curve. The axial sectional area of the probe unit 3 is reduced at several vibration nodes partway in the axial direction so that amplitude necessary for a treatment can be obtained at the probe distal end 3a. Rubber rings formed of an elastic member with a ring shape are attached at several positions of the vibration nodes partway in the axial direction of the probe unit 3. Thus, these rubber rings prevent interference between the probe unit 3 and the sheath unit 5.

A flange portion 14 is provided at the position of the vibration node closest to the side of the proximal end in the axial direction of the probe unit 3. As shown in FIG. 37, keyway-shaped engaging concave portions 15 are formed on the outer peripheral surface of this flange portion 14 at three places in a circumferential direction.

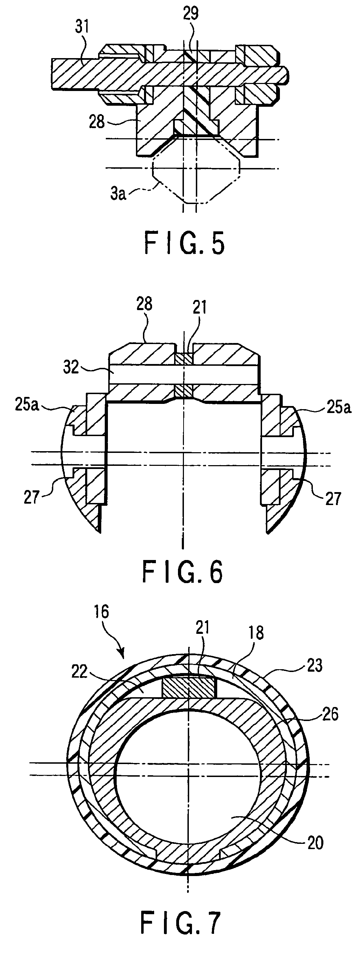

The sheath unit 5 has a sheath main unit 16 formed by a cylindrical member, and a jaw 17 disposed at the distal end of the sheath main unit 16. The sheath main unit 16 has a metal outer cylinder 18 whose sectional shape is circular as shown in FIG. 7, and a metal inner cylinder 19 whose sectional shape is non-circular, for example, D-shaped. A channel 22 for passing a drive shaft 21 of the jaw 17 is formed between the outer cylinder 18 and the inner cylinder 19.

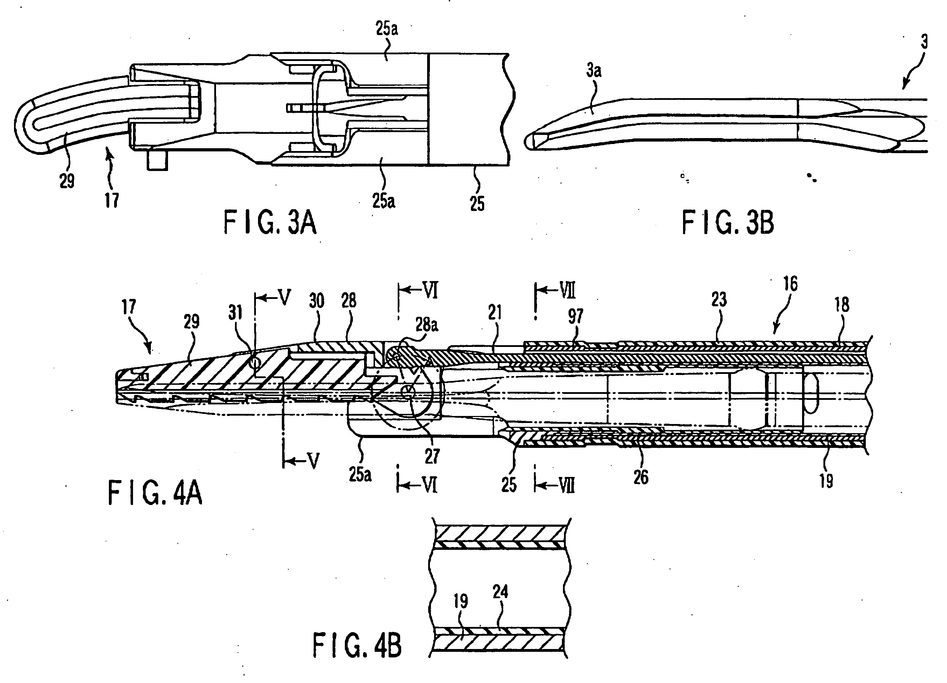

As shown in FIG. 4A, the outer peripheral surface of the outer cylinder 18 is covered with an insulating tube 23. As shown in FIG. 4B, an insulating coating 24 is formed by an insulating material on the inner peripheral surface of the inner cylinder 19. In addition, an insulating tube may be provided on the inner peripheral surface of the inner cylinder 19. Thus, the inner cylinder 19 is electrically insulated from the probe unit 3 by the insulating coating 24.

The proximal end of a substantially cylindrical distal end cover 25 is fixed to the distal end of the outer cylinder 18. On the side of the inner peripheral surface of the proximal end of the distal end cover 25, there is attached a pipe-shaped holding member 26 for holding the probe unit 3 to prevent this probe unit 3 from contacting the distal end cover 25. A channel 20 having a circular section for passing the probe unit 3 is formed inside the holding member 26.

As shown in FIG. 3A, a pair of right and left jaw support portions 25a is provided at the distal end of the distal end cover 25 to extend forward from the outer cylinder 18. A metal jaw main unit 28 of the jaw 17 is swingably attached to these jaw support portions 25a via two supporting point pins 27, as shown in FIG. 6. This jaw 17 is formed to have a substantially J-shaped curve corresponding to the probe distal end 3a of the probe unit 3, as shown in FIG. 3A. Thus, the jaw 17 is opposite to the probe distal end 3a of the probe unit 3 and swingably supported on the two supporting point pins 27 (see FIG. 6). The jaw 17 is operated to swing between an open position at which the jaw 17 swings in a direction to move away from the probe distal end 3a of the probe unit 3 and a closing position at which the jaw 17 swings in a direction to approach the side of the probe distal end 3a of the probe unit 3. If the jaw 17 is operated to swing to the closing position, the living tissue is gripped between the jaw 17 and the probe distal end 3a of the probe unit 3.

The jaw main unit 28 has a grip member 29 made of a resin such as PTFE, and a metal grip member attachment member 30 for holding the grip member 29. The grip member 29 is attached to the grip member attachment member 30 so that this grip member 29 can swing over a given angle by a pin 31 (see FIG. 5). Further, the distal end of the drive shaft 21 is coupled to the rear end of the jaw main unit 28 via a pin 28a, as shown in FIG. 4A. This drive shaft 21 passes inside the distal end cover 25, and then passes between the outer cylinder 18 and the inner cylinder 19 of the sheath main unit 16 as shown in FIG. 7, thus extending to the side of the proximal end of the sheath main unit 16.

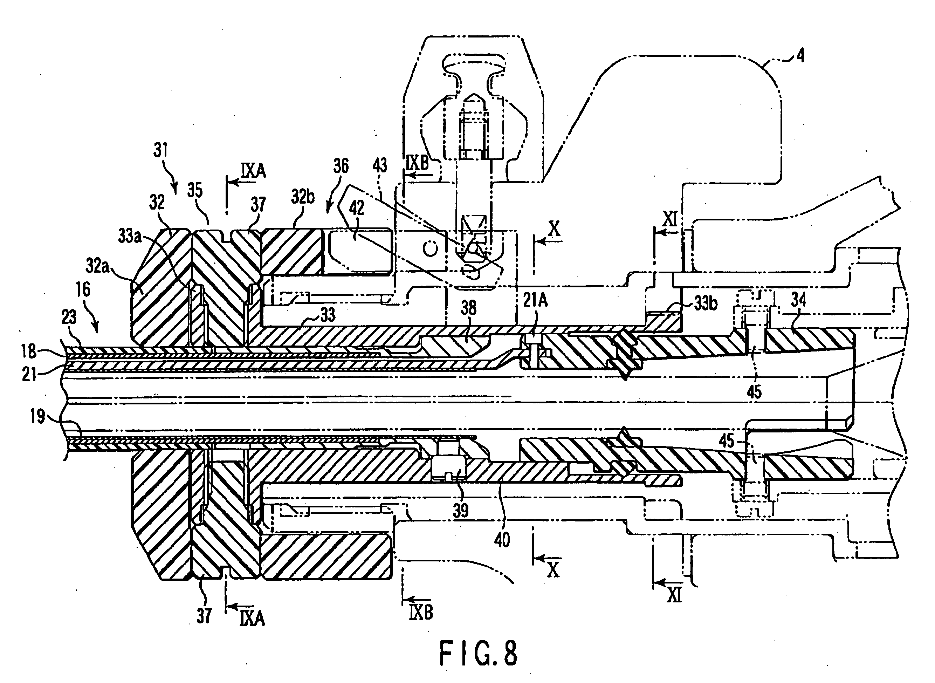

FIG. 8 shows the proximal end of the sheath main unit 16. An attachment/detachment mechanism section 31 for attachment to/detachment from the handle unit 4 is provided at the proximal end of the sheath main unit 16. The attachment/detachment mechanism section 31 has a cylindrical large-diameter pinch member 32 formed of a resin material, a guide cylindrical member 33 formed by a metal cylindrical member, and a cylindrical connecting pipe member 34 formed of a resin material.

The pinch member 32 has a first ring-shaped fixing portion 32a disposed at the front end, and a second cylindrical fixing portion 32b disposed at the rear end. The inner peripheral surface of the first fixing portion 32a is fixed to the outer peripheral surface of the proximal end of the sheath main unit 16. The second fixing portion 32b of the pinch member 32 has a fixing portion 35 of the guide cylindrical member 33 disposed on the front end side, and a portion 36 disposed on the rear end side for attachment to/detachment from the handle unit 4.

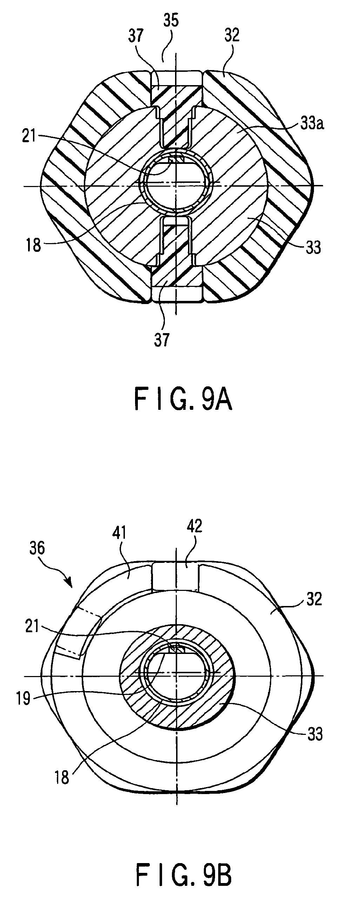

The guide cylindrical member 33 has a large-diameter front end flange portion 33a disposed at the front end, and an outer peripheral flange portion 33b disposed on the rear end side. As shown in FIG. 9A, the front end flange portion 33a of the guide cylindrical member 33 is fixed to the pinch member 32 by two fixing screws 37 made of a resin while being inserted in the pinch member 32.

A metal joining pipe 38 is disposed inside the guide cylindrical member 33. The inner peripheral surface at the front end of this joining pipe 38 is fixed to the outer cylinder 18 of the sheath main unit 16 by laser welding. Further, the joining pipe 38 is fixed to the guide cylindrical member 33 by a metal fixing screw 39. This permits electric conduction between the guide cylindrical member 33, the fixing screw 39, the joining pipe 38, the outer cylinder 18, the distal end cover 25, the supporting point pins 27 and the jaw main unit 28, thereby forming a sheath unit side electric path 40 for transmitting a high-frequency current.

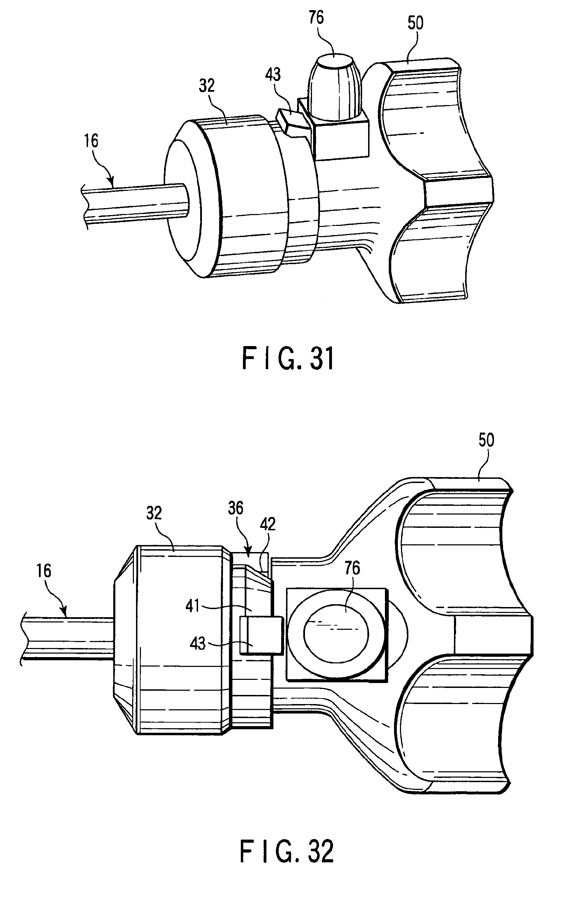

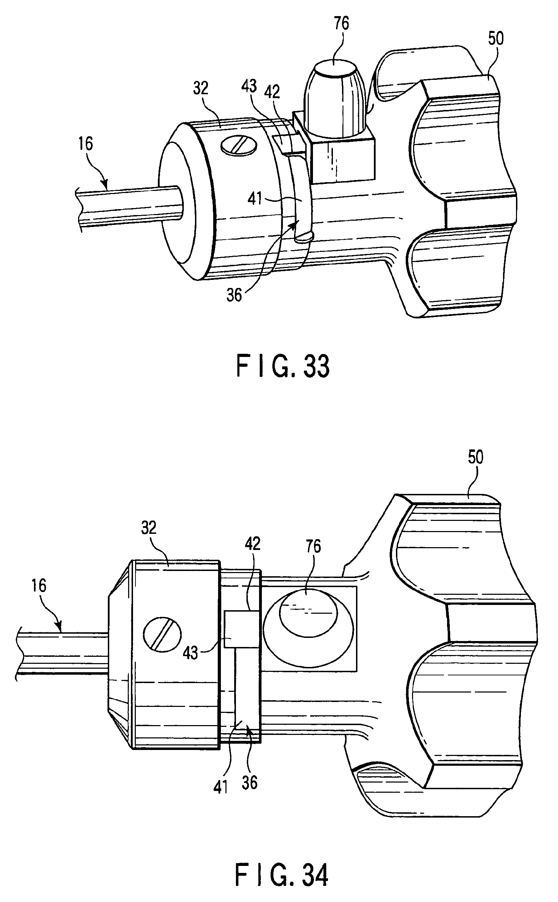

The attachment/detachment portion 36 of the pinch member 32 has a guide groove 41 in the form of an inclined surface provided to extend along a circumferential direction as shown in FIG. 9B, and an engaging concave portion 42 formed at one end of this guide groove 41. The guide groove 41 has a tapered inclined surface whose outside diameter becomes smaller as it approaches the side of the rear end of the pinch member 32. The engaging concave portion 42 is formed by a recessed portion whose diameter is smaller than that of the inclined surface of the guide groove 41. An engaging lever 43 described later on the side of the handle unit 4 removably engages with the engaging concave portion 42. FIGS. 33 and 34 show how the engaging lever 43 engages with the engaging concave portion 42, and FIGS. 31 and 32 show a disengaged state in which the engaging lever 43 is pulled out of the engaging concave portion 42.

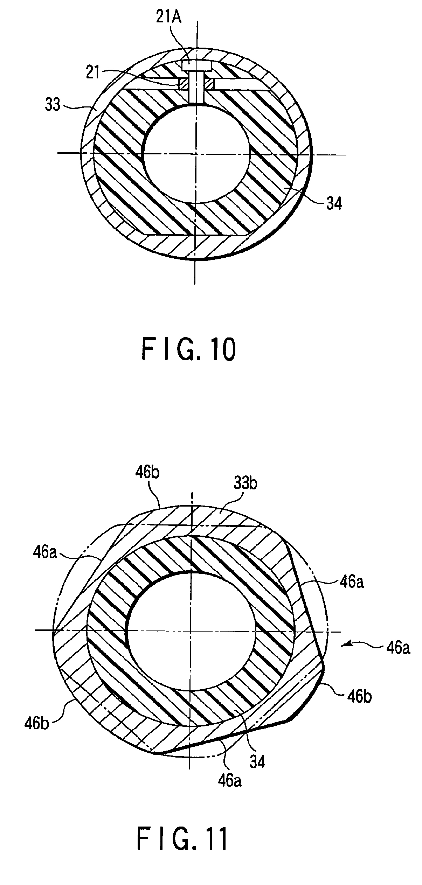

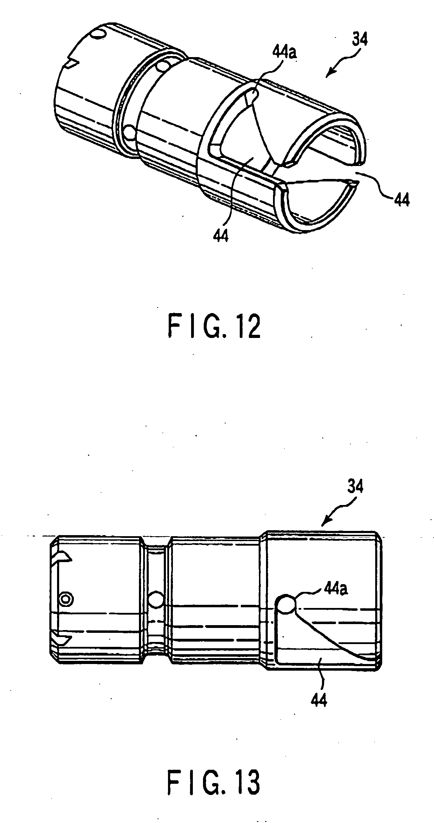

The connecting pipe member 34 is inserted into the guide cylindrical member 33 slidably in a direction of the axis line of the sheath main unit 16. The proximal end of the drive shaft 21 is fixed to the distal end of this connecting pipe member 34 via a pin 21A (see FIG. 10). Two guide grooves 44 shown in FIGS. 12 and 13 are provided at the proximal end of the connecting pipe member 34. Engaging pins 45 described later on the side of the handle unit 4 removably engage with the guide grooves 44. At the terminal end of the guide groove 44, there is formed an engaging groove 44a which regulates the movement of the engaging pin 45 in the direction of the axis line of the sheath main unit 16.

The outer peripheral flange portion 33b has a non-circular engaging portion 46. In the engaging portion 46, there are formed three plane portions 46a formed by cutting off a plurality of places, three places in the present embodiment, in the circular outer peripheral surface of the outer peripheral flange portion 33b. Corner portions 46b whose diameters are larger than those of the plane portions 46a are formed at junctions between the three plane portions 46a. Thus, the engaging portion 46 whose sectional shape is substantially close to a triangular shape is formed in the outer peripheral flange portion 33b. In addition, this non-circular engaging portion 46 does not necessarily have to have the substantially triangular shape, and various shapes including polygonal shapes such as quadrangular and pentangular shapes can be conceived as long as they are non-circular shapes.

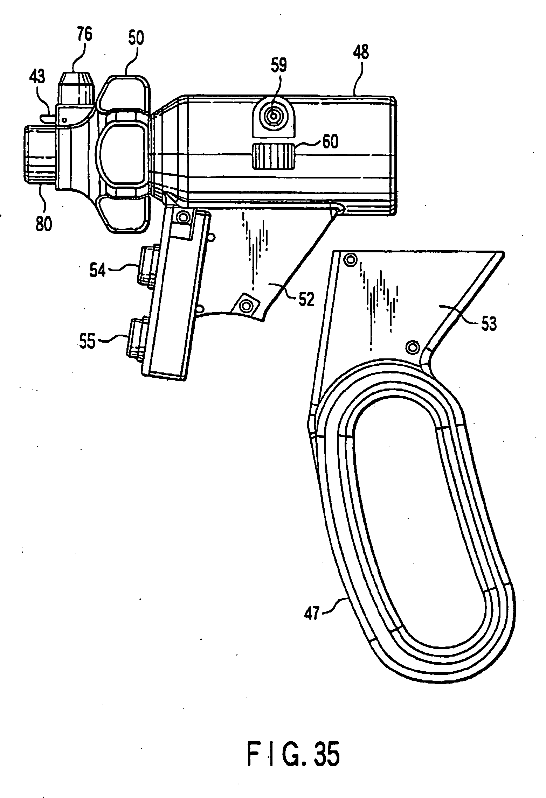

The handle unit 4 mainly has a fixed handle 47, a holding cylinder 48, a movable handle 49, a swing operation knob 50, and a handle unit side electric path 95 for transmitting a high-frequency current. The holding cylinder 48 is disposed on the top of the fixed handle 47. A switch holding portion 51 is provided between the fixed handle 47 and the holding cylinder 48. As shown in FIG. 35, the switch holding portion 51 has a switch attachment portion 52 fixed to the lower end of the holding cylinder 48, and a cover member 53 fixed to the upper end of the fixed handle 47. The switch attachment portion 52 has a plurality of hand switch buttons, in the present embodiment, two hand switch buttons (e.g., a coagulation switch button 54 and an incision switch button 55) which are push button switches. In the switch attachment portion 52, there are incorporated a coagulation switch 54a operated by the coagulation switch button 54, an incision switch 55a operated by the incision switch button 55, and a wiring line circuit board 92. To the wiring line circuit board 92, there are connected a coagulation wiring line 93a whose one end is connected to the coagulation switch 54a, an incision wiring line 93b whose one end is connected to the incision switch 55a, and a ground wiring line 93c whose one end is connected to a ground common terminal. These three wiring lines 93a to 93c are incorporated in the switch holding portion 51 in a rolled state.

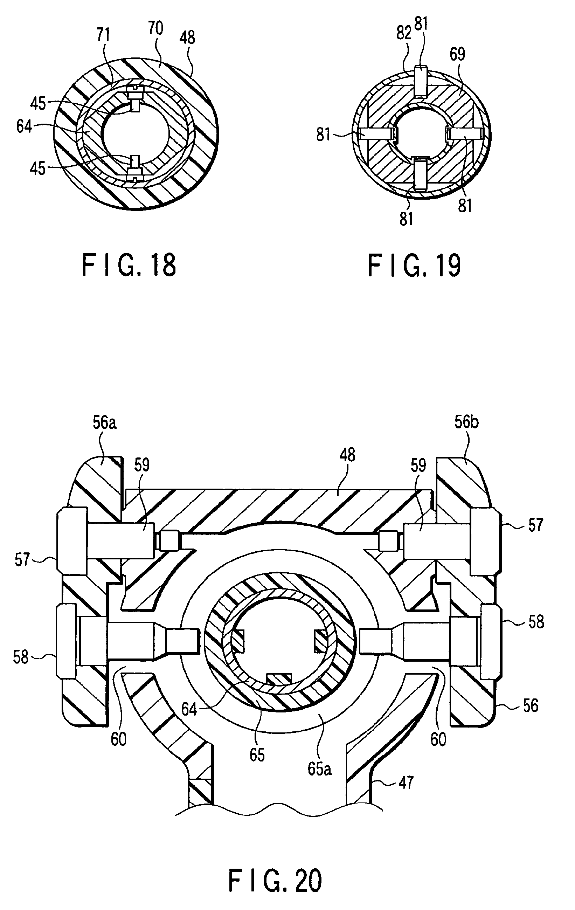

The movable handle 49 has a substantially U-shaped arm portion 56 on its top. The U-shaped arm portion 56 has two arms 56a and 56b, as shown in FIG. 20. The movable handle 49 is set to the holding cylinder 48 so that the holding cylinder 48 is inserted between the two arms 56a and 56b.

Each of the arms 56a and 56b has a supporting point pin 57 and an action pin 58. Pin receiving holes 59 and windows 60 are formed on both sides of the holding cylinder 48. The supporting point pin 57 of each of the arms 56a and 56b is inserted in the pin receiving hole 59 of the holding cylinder 48. Thus, the upper end of the movable handle 49 is swingably supported on the holding cylinder 48 via the supporting point pins 57.

Finger hooks 61 and 62 are provided at lower ends of the fixed handle 47 and the movable handle 49, respectively. Thus, the handles are gripped by fingers put on the finger hooks, such that the movable handle 49 swings via the supporting point pins 57, and the movable handle 49 opens/closes with respect to the fixed handle 47.

Each of the action pins 58 of the movable handle 49 extends into the holding cylinder 48 through the window 60 of the holding cylinder 48. An operation force transmitting mechanism 63 for transmitting the operation force of the movable handle 49 to the drive shaft 21 of the jaw 17 is provided inside the holding cylinder 48.

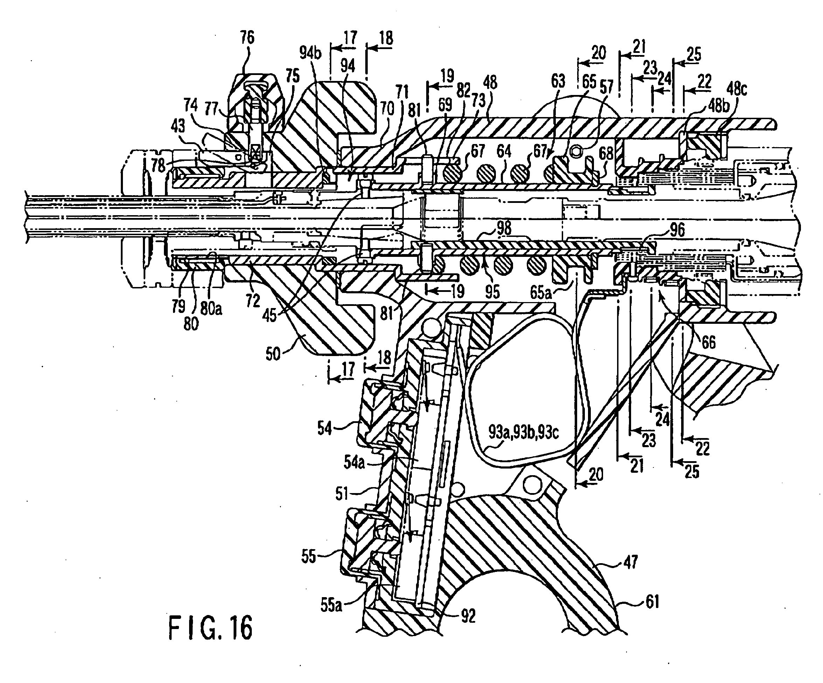

As shown in FIG. 16, the operation force transmitting mechanism 63 has a cylindrical spring bearing member 64 mainly made of a metal, and a slider member 65 made of a resin. The spring bearing member 64 is disposed coaxially with the central line of the holding cylinder 48, and provided to extend in the same direction as the insertion direction of the probe unit 3.

The proximal end of the spring bearing member 64 is coupled to a later-described cylindrical contact unit 66 fixed to the proximal end of the holding cylinder 48 to be able to swing in a direction around the axis thereof and to be able to move back and forth in the same direction as the insertion direction of the probe unit 3. The pair of engaging pins 45 on the side of the handle unit 4 described above is provided to inwardly protrude at the distal end of the spring bearing member 64. When the handle unit 4 is coupled to the sheath unit 5, the pair of engaging pins 45 on the side of the handle unit 4 removably engages with the engaging grooves 44a at the terminal end of the guide grooves 44 of the sheath unit 5.

On the outer peripheral surface of the spring bearing member 64, there are provided a coil spring 67, the slider member 65, a stopper 68 and a spring bearing 69. The front end of the coil spring 67 is fixed to the spring bearing 69. The stopper 68 regulates the moving position of the rear end side of the slider member 65. The coil spring 67 is installed between the spring bearing 69 and the slider member 65 with a given amount of force of equipment.

A ring-shaped engaging groove 65a is formed on the outer peripheral surface of the slider member 65 along a circumferential direction. The action pins 58 of the movable handle 49 engage with the engaging groove 65a so that they are inserted in this engaging groove 65a, as shown in FIG. 20. Thus, when the movable handle 49 is gripped to close the movable handle 49 with respect to the fixed handle 47, the movable handle 49 swings so that the action pins 58 swing around the supporting point pins 57. The slider member 65 interlocked with the swing operation of the supporting point pins 57 moves forward along the axial direction. At this point, the spring bearing member 64 coupled to the slider member 65 via the coil spring 67 also moves back and forth together with the slider member 65. Thus, the operation force of the movable handle 49 is transmitted to the connecting pipe member 34 via the pair of engaging pins 45, and the drive shaft 21 of the jaw 17 moves forward. Therefore, the jaw main unit 28 of the jaw 17 swings via the supporting point pins 27.

Furthermore, when the living tissue is gripped between the grip member 29 of the jaw 17 and the probe distal end 3a of the probe unit 3 in accordance with the above operation, the grip member 29 swings at a given angle on the pin 31 to follow the bending of the probe distal end 3a so that force is equally applied to the overall length of the grip member 29. When the ultrasonic waves are output in this state, it is possible to coagulate or incise the living tissue such as a blood vessel.

A ring-shaped bearing 70 is formed at the front end of the holding cylinder 48. A cylindrical rotation transmitting member 71 made of a metal is coupled to the bearing 70 swingably in a direction around the axis. In the rotation transmitting member 71, there are formed a protrusion 72 protruding ahead of the bearing 70, and a large-diameter portion 73 provided to extend from the bearing 70 onto the internal side of the holding cylinder 48.

The swing operation knob 50 is fixed to the protrusion 72 in an externally fitted state. The engaging lever 43 is provided at the front end of this swing operation knob 50. The intermediate portion of the engaging lever 43 is swingably coupled to the protrusion 72 via a pin 74. The proximal end of the engaging lever 43 extends into the inside of a lever receiving concave portion 75 formed in the front surface of the swing operation knob 50.

An operation button 76 for operating the engaging lever 43 in a disengaging direction is provided on the outer peripheral surface at the front end of the swing operation knob 50. A downward actuating pin 77 is provided to protrude in the operation button 76. The actuating pin 77 extends onto the internal side of the lever receiving concave portion 75 via a wall hole of the swing operation knob 50. The proximal end of the engaging lever 43 is coupled to the lower end of the actuating pin 77 via a pin 78.

A drop preventing ring 80 for the swing operation knob 50 is provided at the distal end of the protrusion 72. A male screw 79 is formed at the distal end of the protrusion 72. A female screw 80a to which the male screw 79 is threadably attached is formed on the inner peripheral surface of the drop preventing ring 80. Thus, the female screw 80a of the drop preventing ring 80 is screwed to the male screw 79 of the protrusion 72, such that the swing operation knob 50 is fixed to the rotation transmitting member 71.

As shown in FIG. 19, four positioning pins 81 made of a metal are provided to diametrically outwardly protrude in the spring bearing 69 of the spring bearing member 64. A long-hole-shaped engaging hole 82 into which one pin 81 of the spring bearing member 64 is formed in the large-diameter portion 73 of the rotation transmitting member 71. The engaging hole 82 is provided to extend in the same direction as the insertion direction of the probe unit 3. Thus, the pin 81 is moved along the engaging hole 82 during the operation of the movable handle 49, thereby preventing the back-and-forth movement of the spring bearing member 64 from being transmitted to the rotation transmitting member 71.

On the contrary, the rotational operation of the rotation transmitting member 71 rotating together with the swing operation knob 50 is transmitted to the side of the spring bearing member 64 via the pin 81 during the rotational operation of the swing operation knob 50. Thus, during the rotational operation of the swing operation knob 50, a set unit including the rotation transmitting member 71, the pin 81, the spring bearing member 64, the slider member 65 and the coil spring 67 inside the holding cylinder 48 is driven to integrally rotate in a direction around the axis together with the swing operation knob 50.

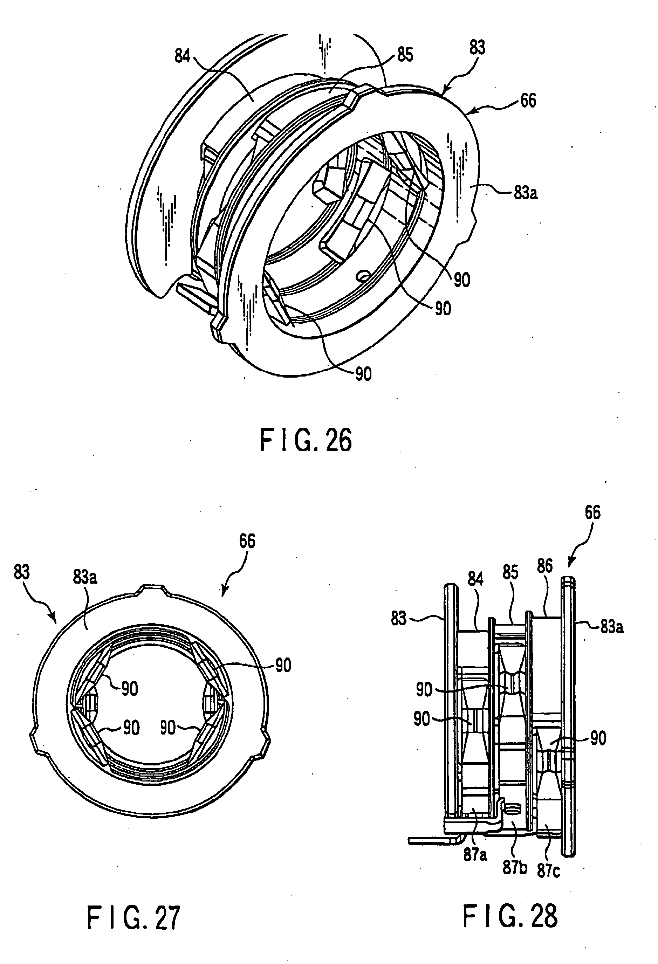

FIGS. 26 to 28 show the cylindrical contact unit 66. The contact unit 66 has a cylindrical electrode holding member 83 made of a resin. The electrode holding member 83 has three (first to third) electrode receiving portions 84, 85 and 86 different in the size of outside diameter, as shown in FIG. 28. The first electrode receiving portion 84 on the distal end side has the smallest diameter, and the third electrode receiving portion 86 on the rear end side has the largest diameter.

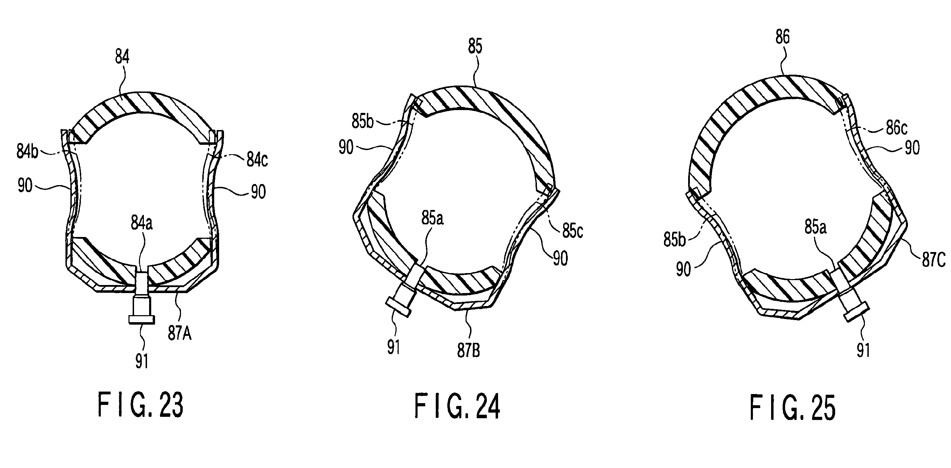

As shown in FIG. 23, the first electrode receiving portion 84 has one contact member fixing hole 84a and two through-holes 84b and 84c. The central lines of the two through-holes 84b and 84c are disposed at positions perpendicular to the central line of the contact member fixing hole 84a.

In the same manner, the second electrode receiving portion 85 has one contact member fixing hole 85a and two through-holes 85b and 85c, as shown in FIG. 24. The third electrode receiving portion 86 has one contact member fixing hole 86a and two through-holes 86b and 86c, as shown in FIG. 25.

The contact member fixing hole 84a of the first electrode receiving portion 84, the contact member fixing hole 85a of the second electrode receiving portion 85 and the contact member fixing hole 86a of the third electrode receiving portion 86 are positioned so that they are displaced from each other in the circumferential direction of the electrode holding member 83.

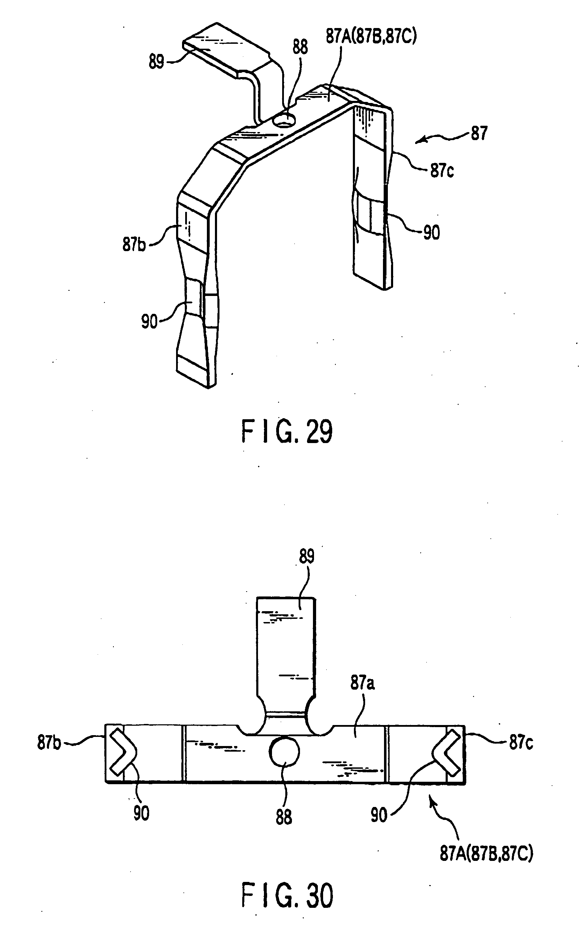

FIGS. 29 and 30 show electrode members 87A, 87B and 87C to be set to the first to third electrode receiving portions 84, 85 and 86. These electrode members 87A, 87B and 87C are formed to have the same shape. Here, the electrode member 87A to be set to the first electrode receiving portion 84 alone will be described, and the same signs are assigned to the same parts of the other electrode members 87B and 87C of the second and third electrode receiving portions 85 and 86, so that the electrode members 87B and 87C will not be described.

The electrode member 87A has one linear fixed portion 87a, and two bending portions 87b and 87c. The one bending portion 87b is disposed at one end of the linear fixed portion 87a, and the other bending portion 87c is disposed at the other end thereof. Thus, the electrode member 87A is formed to be bent into a substantially U shape, as shown in FIG. 29.

A hole 88 and an L-shaped wiring line connecting portion 89 are provided at the central position of the fixed portion 87a. Constricted portions 90 having an inwardly curving shape are formed in the two bending portions 87b and 87c at their central positions.

When the electrode member 87A is set to the first electrode receiving portion 84, a fixing pin 91 is inserted into the hole 88 of the fixed portion 87a of the electrode member 87A and into the contact member fixing hole 85a of the first electrode receiving portion 84. The electrode member 87A is fixed to the first electrode receiving portion 84 by the fixing pin 91. At this point, the constricted portion 90 of the one bending portion 87b of the electrode member 87A is disposed to be inserted into the one through-hole 85b of the first electrode receiving portion 84, while the constricted portion 90 of the other bending portion 87c of the electrode member 87A is disposed to be inserted into the other through-hole 85c. The same holds true for the case where the electrode member 87B is set to the second electrode receiving portion 85 and for the case where the electrode member 87C is set to the third electrode receiving portion 86.

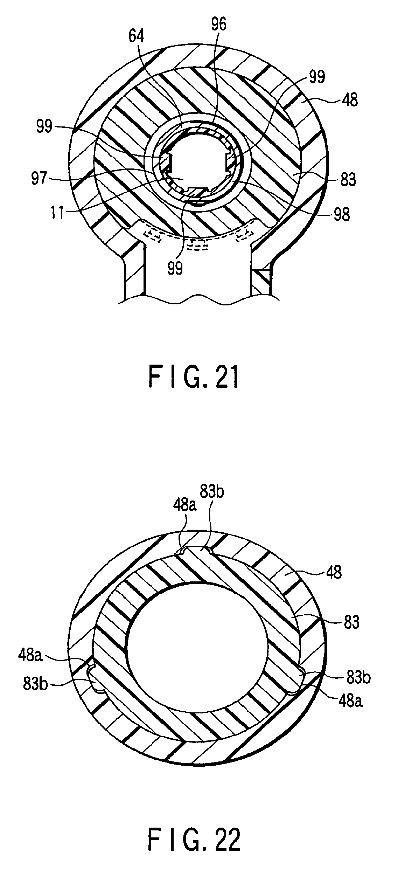

As shown in FIG. 22, a large-diameter fixed flange portion 83a is formed at the rear end of the electrode holding member 83 of the contact unit 66. Engaging convex portions 83b are provided to protrude on the outer peripheral surface of the fixed flange portion 83a at a plurality of places, in the present embodiment, at three places. Engaging concave portions 48a are formed on the inner peripheral surface at the rear end of the holding cylinder 48 at positions corresponding to the three engaging convex portions 83b of the fixed flange portion 83a. When the electrode holding member 83 is set to the holding cylinder 48, they are engaged with and fixed to each other so that the three engaging convex portions 83b of the fixed flange portion 83a are inserted into the engaging concave portions 48a of the holding cylinder 48. This regulates the rotation of the electrode holding member 83 with respect to the holding cylinder 48 in the direction around the axis.

A step portion 43b for contacting the fixed flange portion 83a of the electrode holding member 83 is formed in the holding cylinder 48. The electrode holding member 83 is screwed to the holding cylinder 48 by a fixing screw 48c so that the fixed flange portion 83a of the electrode holding member 83 is placed in collision with this step portion 43b. This regulates the axial movement of the electrode holding member 83 with respect to the holding cylinder 48.

The ends of three wiring lines 93a to 93c incorporated in the switch holding portion 51 are connected to the wiring line connecting portions 89 of the three electrode members 87A, 87B and 87C set to the contact unit 66.

The contact unit 66 is further provided with a substantially C-shaped electric contact member 96 configured by a metal leaf spring, as shown in FIG. 21. The electric contact member 96 is connected to the outer peripheral surface at the proximal end of the spring bearing member 64.

The handle unit side electric path 95 comprises the electric contact member 96, the spring bearing member 64, the positioning pins 81 and the rotation transmitting member 71.

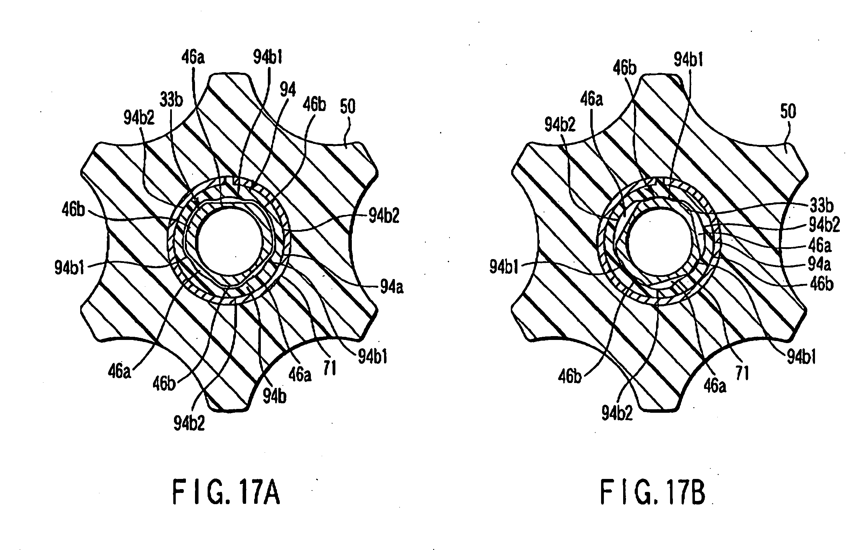

On the inner peripheral surface of the rotation transmitting member 71, there is provided engaging means 94 for removably engaging with the outer peripheral flange portion 33b of the sheath unit 5 substantially at the central position along the axial direction. As shown in FIGS. 17A and 17B, this engaging means 94 has an insertion hole 94a into which the outer peripheral flange portion 33b is inserted when the sheath unit 5 is coupled to the handle unit 4, and a conductive rubber ring (urging means) 94b disposed in the insertion hole 94a.

The shape of the inner peripheral surface of the conductive rubber ring 94b is substantially the same as that of the engaging portion 46 of the outer peripheral flange portion 33b. In other words, there are formed three plane portions 94b1 cut at a plurality of places, in the present embodiment, at three places on the circular inner peripheral surface, and three corner portions 94b2 which are disposed at junctions between the three plane portions 94b1 and which have diameters larger than those of the plane portions 94b1. This forms a sectional shape substantially close to a triangular shape. Therefore, the conductive rubber ring 94b is held at a non-compression position where it is in a natural state, at a position where the shape of the inner peripheral surface of the conductive rubber ring 94b corresponds to the engaging portion 46 of the outer peripheral flange portion 33b, that is, in a situation where the three corner portions 46b of the outer peripheral flange portion 33b correspond to the three corner portions 94b2 of the conductive rubber ring 94b, as shown in FIG. 17A. On the contrary, if the handle unit 4 and the sheath unit 5 are rotated relatively to each other in the direction around the central axis of the sheath unit 5, the conductive rubber ring 94b is switched to a pressure-contact position at which the conductive rubber ring 94b is brought into pressure-contact with the three corner portions 46b of the outer peripheral flange portion 33b, as shown in FIG. 17B. At this point, the three corner portions 46b of the outer peripheral flange portion 33b contact the three plane portions 94b1 of the conductive rubber ring 94b, and are thus compressed.

In the present embodiment, the conductive rubber ring 94b is held at the non-compression position where it is in the natural state as shown in FIG. 17A during an insertion operation (see FIGS. 31 and 32) in which the outer peripheral flange portion 33b of the sheath unit 5 is inserted straight into the conductive rubber ring 94b when the sheath unit 5 is coupled to the handle unit 4. At this point, the engaging lever 43 on the side of the handle unit 4 is held while being stranded on the inclined surface of the guide groove 41 of the pinch member 32 of the sheath unit 5. Then, the pinch member 32 of the sheath unit 5 is rotated with respect to the handle unit 4 in a direction around the axis, such that the engaging lever 43 on the side of the handle unit 4 engages in an inserted state with the engaging concave portion 42 at one end of the guide groove 41, as shown in FIGS. 33 and 34. At this point, the conductive rubber ring 94b is switched to a pressure-contact position at which the conductive rubber ring 94b is brought into pressure-contact with the three corner portions 46b of the outer peripheral flange portion 33b, as shown in FIG. 17B. This permits conduction, via the conductive rubber ring 94b, between the sheath unit side electric path 40 (formed between the guide cylindrical member 33, the fixing screw 39, the joining pipe 38, the outer cylinder 18, the distal end cover 25, the supporting point pins 27 and the jaw main unit 28) and the handle unit side electric path 95 (formed between the electric contact member 96, the spring bearing member 64, the positioning pins 81 and the rotation transmitting member 71). At this point, a second high-frequency electric path 97 for transmitting a high-frequency current is formed in a combination of the sheath unit 5 and the handle unit 4.

As shown in FIG. 21, the handle unit 4 has a tubular member 98 formed by an insulating material on the inner peripheral surface of the spring bearing member 64. The tubular member 98 is fixed to the inner peripheral surface of the spring bearing member 64. Thus, the tubular member 98 provides insulation between the first high-frequency electric path 13 and the second high-frequency electric path 97 when the probe unit 3 is connected to the handle unit 4.

On the inner peripheral surface of the tubular member 98, there are formed three engaging convex portions 99 corresponding to the three engaging concave portions 15 (see FIG. 37) of the flange portion 14 of the probe unit 3. When the probe unit 3 is connected to the handle unit 4, the three engaging convex portions 99 of the tubular member 98 removably engage with the three engaging concave portions 15 of the flange portion 14 of the probe unit 3. This regulates the positions of the probe unit 3 and the tubular member 98 of the handle unit 4 in the rotation direction. Thus, a combination of the probe unit 3 and the transducer unit 2 is driven to integrally rotate together with a set unit inside the holding cylinder 48 during the rotational operation of the swing operation knob 50.

In addition, the engaging portion between the flange portion 14 of the probe unit 3 and the tubular member 98 is not limited to the configuration described above. For example, the tubular member 98 may be formed to have a D-shaped section, and the flange portion 14 of the probe unit 3 may be formed to have a D-shaped section correspondingly.

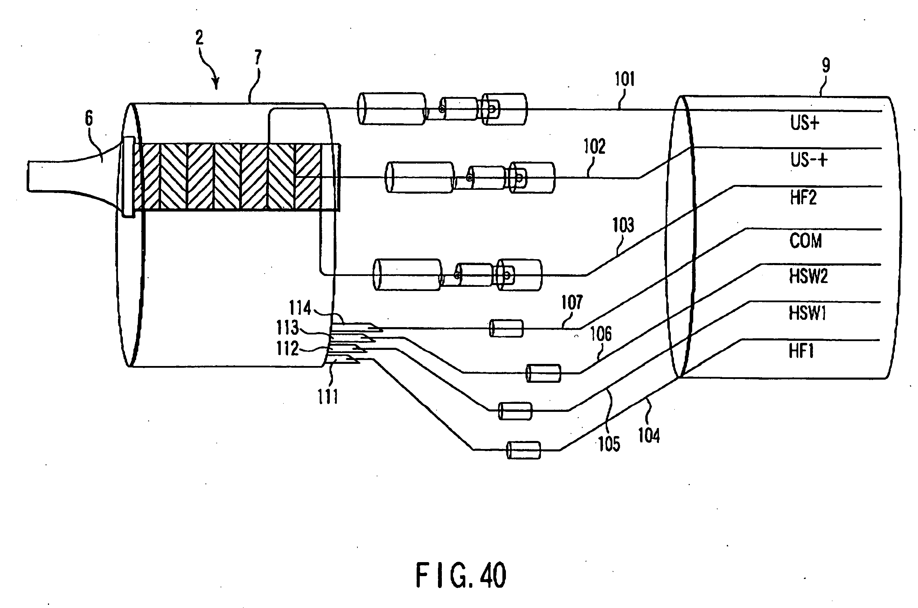

The front end of the transducer unit 2 is removably coupled to the contact unit 66. In one cable 9 at the rear end of the transducer unit 2, there are incorporated two wiring lines 101 and 102 for the ultrasonic transducer, two wiring lines 103 and 104 for high-frequency conduction, and three wiring lines 105, 106 and 107 connected to the wiring line circuit board 92 within the switch holding portion 51, as shown in FIG. 40. The distal ends of the two wiring lines 101 and 102 for the ultrasonic transducer are connected to the ultrasonic transducer 6. The distal end of the one wiring line 103 for the high-frequency conduction is connected to the ultrasonic transducer 6.

Four first to fourth conducting plates 111 to 114 for electric connection are provided at the rear end of the transducer unit 2. The distal end of the other wiring line 104 for the high-frequency conduction is connected to the first conducting plate 111. The three wiring lines 105, 106 and 107 are connected to the second to fourth conducting plates 112 to 114, respectively.

FIG. 41 shows an internal configuration of the front end of the transducer unit 2. A connection cylindrical portion 121 is formed at the distal end of the transducer cover 7. A leaf-spring-shaped C ring 122 in which a part of a ring is cut off is attached onto the outer peripheral surface of the connection cylindrical portion 121. Three steps of (first to third) cylindrical portions 123 to 125 with differently dimensioned outside diameters are provided to protrude on the inner side of the connection cylindrical portion 121. The first cylindrical portion 123 has the smallest outside diameter, and the largest length of protrusion from the distal end of the connection cylindrical portion 121. The second cylindrical portion 124 has an outside diameter larger than that of the first cylindrical portion 123, and the length of its protrusion from the distal end of the connection cylindrical portion 121 is smaller than that of the first cylindrical portion 123. The third cylindrical portion 125 has the largest outside diameter, and the length of its protrusion from the distal end of the connection cylindrical portion 121 is smaller than that of the second cylindrical portion 124.

A cylindrical first contact member 131 is attached onto the outer peripheral surface of the first cylindrical portion 123. In the same manner, a cylindrical second contact member 132 is attached onto the outer peripheral surface of the second cylindrical portion 124, and a cylindrical third contact member 133 is attached onto the outer peripheral surface of the third cylindrical portion 125. The second conducting plate 112 is connected to the first contact member 131, the third conducting plate 113 is connected to the second contact member 132, and the fourth conducting plate 114 is connected to the third contact member 133.

A cylindrical fourth contact member 134 is attached onto the inner peripheral surface of the first cylindrical portion 123. The fourth contact member 134 is connected to the first conducting plate 111.

When the handle unit 4 is coupled to the transducer unit 2, the contact unit 66 of the handle unit 4 is connected to the front end of the transducer unit 2. At this point, the electrode member 87A of the contact unit 66 is connected to the first contact member 131 of the transducer unit 2. At the same time, the electrode member 87B of the contact unit 66 is connected to the second contact member 132 of the transducer unit 2, the electrode member 87C of the contact unit 66 is connected to the third contact member 133 of the transducer unit 2, and the C-shaped electric contact member 96 of the contact unit 66 is connected to the fourth contact member 134 of the transducer unit 2.

Next, effects of the present embodiment will be described. In the hand piece 1 of the ultrasonic operating apparatus of the present embodiment, the four units including the transducer unit 2, the probe unit 3, the handle unit 4 and the sheath unit 5 are detachable, as shown in FIG. 2. During the use of the hand piece 1, the transducer unit 2 is coupled to the probe unit 3. Thus, the first high-frequency electric path 13 for transmitting the high-frequency current is formed in the combination of the transducer unit 2 and the probe unit 3.

Subsequently, the handle unit 4 is coupled to the sheath unit 5. When the handle unit 4 is coupled to the sheath unit 5, the connecting pipe member 34 is inserted into the rotation transmitting member 71 of the handle unit 4 while the pinch member 32 of the sheath unit 5 is being gripped. When the sheath unit 5 is coupled to the handle unit 4, the engaging lever 43 on the side of the handle unit 4 is held while being stranded on the inclined surface of the guide groove 41 of the pinch member 32 of the sheath unit 5, as shown in FIGS. 31 and 32. At this point, as shown in FIG. 17A, the engaging lever 43 is held at the position where the shape of the inner peripheral surface of the conductive rubber ring 94b corresponds to the engaging portion 46 of the outer peripheral flange portion 33b, that is, in a situation where the three corner portions 46b of the outer peripheral flange portion 33b correspond to the three corner portions 94b2 of the conductive rubber ring 94b. Therefore, the outer peripheral flange portion 33b of the sheath unit 5 is inserted straight into the conductive rubber ring 94b. During this insertion operation, the conductive rubber ring 94b is held at the non-compression position where it is in the natural state, as shown in FIG. 17A. In this state, there is no conduction between the sheath unit side electric path 40 and the handle unit side electric path 95.

Then, after this insertion operation is finished, the pinch member 32 of the sheath unit 5 is rotated in the direction around the axis with respect to the handle unit 4. Owing to this operation, the engaging lever 43 on the side of the handle unit 4 engages in an inserted state with the engaging concave portion 42 at one end of the guide groove 41, as shown in FIGS. 33 and 34. At this point, the conductive rubber ring 94b is switched to the pressure-contact position at which the conductive rubber ring 94b is placed in pressure-contact with the three corner portions 46b of the outer peripheral flange portion 33b, as shown in FIG. 17B. This permits conduction, via the conductive rubber ring 94b, between the sheath unit side electric path 40 and the handle unit side electric path 95. As a result, the second high-frequency electric path 97 for transmitting a high-frequency current is formed in the combination of the sheath unit 5 and the handle unit 4.

During this rotational operation of the sheath unit 5 in a direction around the axis, the pair of engaging pins 45 on the side of the handle unit 4 removably engages with the engaging grooves 44a at the terminal end of the guide grooves 44 of the sheath unit 5 at the same time. Thus, the spring bearing member 64 on the side of the handle unit 4 is coupled to the connecting pipe member 34 on the side of the sheath unit 5 via the engaging pins 45. As a result, the operation force on the side of the handle unit 4 during the operation of closing the movable handle 49 with respect to the fixed handle 47 can be transmitted to the drive shaft 21 of the jaw 17 on the side of the sheath unit 5. This is the state where the sheath unit 5 is coupled to the handle unit 4.

Subsequently, the combination of the sheath unit 5 and the handle unit 4 and the combination of the ultrasonic transducer 6 and the probe unit 3 are set to be united into one. During this setting operation, the contact unit 66 of the handle unit 4 is connected to the front end of the transducer unit 2. At this point, the electrode member 87A of the contact unit 66 is connected to the first contact member 131 of the transducer unit 2. At the same time, the electrode member 87B of the contact unit 66 is connected to the second contact member 132 of the transducer unit 2, the electrode member 87C of the contact unit 66 is connected to the third contact member 133 of the transducer unit 2, and the C-shaped electric contact member 96 of the contact unit 66 is connected to the fourth contact member 134 of the transducer unit 2. Thus, the second high-frequency electric path 97 of the combination of the sheath unit 5 and the handle unit 4 is connected to the wiring line 104 for the high-frequency conduction within the cable 9. Further, the three wiring lines 105, 106 and 107 within the cable 9 are connected to the wiring line circuit board 92 within the switch holding portion 51. This is the state where the setting of the hand piece 1 is finished.

Then, during the use of this hand piece 1, the movable handle 49 is closed with respect to the fixed handle 47, such that the drive shaft 21 is axially moved in conjunction with the operation of this movable handle 49, and the jaw 17 is driven to open/close with respect to the probe distal end 3a of the probe unit 3 in conjunction with the axial back-and-forth movement of the drive shaft 21. Thus, the living tissue is gripped between the jaw 17 and the probe distal end 3a of the probe unit 3.

In this state, one of the coagulation switch button 54 and the incision switch button 55 of the fixed handle 47 is selectively pushed. When the coagulation switch button 54 is pushed, electricity is conducted in the first high-frequency electric path 13 for conducting a high-frequency current to the probe distal end 3a of the probe unit 3 and in the second high-frequency electric path 97 for conducting a high-frequency current to the jaw main unit 28 of the sheath unit 5. Thus, two bipolar electrodes for the high-frequency treatment are formed by the probe distal end 3a of the probe unit 3 and the jaw main unit 28 of the sheath unit 5. Then, the high-frequency current is conducted across the two bipolar electrodes formed by the probe distal end 3a of the probe unit 3 and the jaw main unit 28 of the sheath unit 5, such that the living tissue between the jaw 17 and the probe distal end 3a of the probe unit 3 can be subjected to the high-frequency treatment by the bipolar.

When the incision switch button 55 is pushed, a drive current is conducted to the ultrasonic transducer 6 simultaneously with the high frequency conduction, and the ultrasonic transducer 6 is driven. Thus, the ultrasonic vibrations from the ultrasonic transducer 6 are transmitted to the probe distal end 3a via the vibration transmitting member 11, such that the treatment such as the incision or removal of the living tissue can be administered using the ultrasonic waves simultaneously with the high frequency conduction. In addition, the ultrasonic waves can also be used to coagulate the living tissue.

Furthermore, during the rotational operation of the swing operation knob 50, the rotational operation of the rotation transmitting member 71 which rotates together with the swing operation knob 50 is transmitted to the side of the spring bearing member 64 via the pins 81. Thus, during the rotational operation of the swing operation knob 50, the set unit of the rotation transmitting member 71, the pins 81, the spring bearing member 64, the slider member 65 and the coil spring 67 within the holding cylinder 48 is driven to integrally rotate in a direction around the axis together with the swing operation knob 50. Moreover, the rotational operation force of the swing operation knob 50 is transmitted to the vibration transmitting member 11 of the probe unit 3 via the tubular member 98 which rotates together with the spring bearing member 64 within the holding cylinder 48. Thus, the set unit within the holding cylinder 48 and the combination of the transducer unit 2 and the probe unit 3 are driven to integrally rotate together in a direction around the axis.

Therefore, the configuration described above provides the following advantages: in the hand piece 1 of the ultrasonic operating apparatus of the present embodiment, the substantially triangular engaging portion 46 is provided in the front end flange portion 33a of the guide cylindrical member 33 of the sheath unit 5. The conductive rubber ring 94b is provided on the inner peripheral surface of the rotation transmitting member 71. Further, when the sheath unit 5 is coupled to the handle unit 4, the pinch member 32 of the sheath unit 5 is rotated in the direction around the axis with respect to the handle unit 4, the conductive rubber ring 94b is switched to the pressure-contact position at which the conductive rubber ring 94b is placed in pressure-contact with the three corner portions 46b of the outer peripheral flange portion 33b, as shown in FIG. 17B. Thus, conduction is permitted, via the pressure-contact portion of the conductive rubber ring 94b, between the sheath unit side electric path 40 and the handle unit side electric path 95, such that the conduction between the sheath unit side electric path 40 and the handle unit side electric path 95 can be stabilized. Therefore, when the handle unit 4 and the sheath unit 5 of the hand piece 1 capable of the bipolar high-frequency treatment are detachably configured, it is possible to prevent the instability of the conduction between the sheath unit side electric path 40 and the handle unit side electric path 95.

Furthermore, since the tubular member 98 is provided in the handle unit 4, the tubular member 98 can provide insulation between the first high-frequency electric path 13 and the second high-frequency electric path 97 when the probe unit 3 is connected to the handle unit 4. When the probe unit 3 is connected to the handle unit 4, the three engaging convex portions 99 of the tubular member 98 removably engage with the three engaging concave portions 15 of the flange portion 14 of the probe unit 3. This regulates the positions of the probe unit 3 and the tubular member 98 of the handle unit 4 in the rotation direction. Thus, the combination of the probe unit 3 and the transducer unit 2 can be driven to integrally rotate together with the set unit inside the holding cylinder 48 during the rotational operation of the swing operation knob 50. In this manner, the tubular member 98 doubles as means for regulating the positions of the probe unit 3 and the tubular member 98 of the handle unit 4 in the rotation direction, so that the number of components in the handle unit 4 can be reduced.

Furthermore, since the sheath unit 5 is detachably coupled to the handle unit 4, the sheath unit 5 alone can be replaced, for example, when the grip member 29 of the jaw 17 at the distal end of the sheath unit 5 is worn. Therefore, costs can be lower than when all the setting components of the handle unit 4 and the sheath unit 5 are replaced as in the case where the sheath unit 5 is integrally set to the handle unit 4.

In the configuration of the hand piece 1 of the ultrasonic operating apparatus of the present embodiment, there are incorporated, in one cable 9 at the rear end of the transducer unit 2, the two wiring lines 101 and 102 for the ultrasonic transducer, the two wiring lines 103 and 104 for high-frequency conduction, and the three wiring lines 105, 106 and 107 connected to the wiring line circuit board 92 within the switch holding portion 51, as shown in FIG. 40. It is therefore not necessary to connect a plurality of cables to the hand piece 1, so that the operability of the hand piece 1 can be enhanced.

Furthermore, in the present embodiment, the switch holding portion 51 is provided in the fixed handle 47, and the coagulation switch button 54 and the incision switch button 55 are incorporated in the fixed handle 47. The connection wiring lines of the coagulation switch button 54 and the incision switch button 55 are disposed within the hand piece 1, and connected to the three wiring lines 105, 106 and 107 incorporated in one cable 9 at the rear end of the transducer unit 2. Therefore, the connection wiring lines for the coagulation switch button 54 and the incision switch button 55 are not coupled to the hand piece 1, for example, as in the case where the coagulation switch button 54 and the incision switch button 55 are externally attached to the hand piece 1. As a result, the number of connecting cords connected to the hand piece 1 can be further reduced.

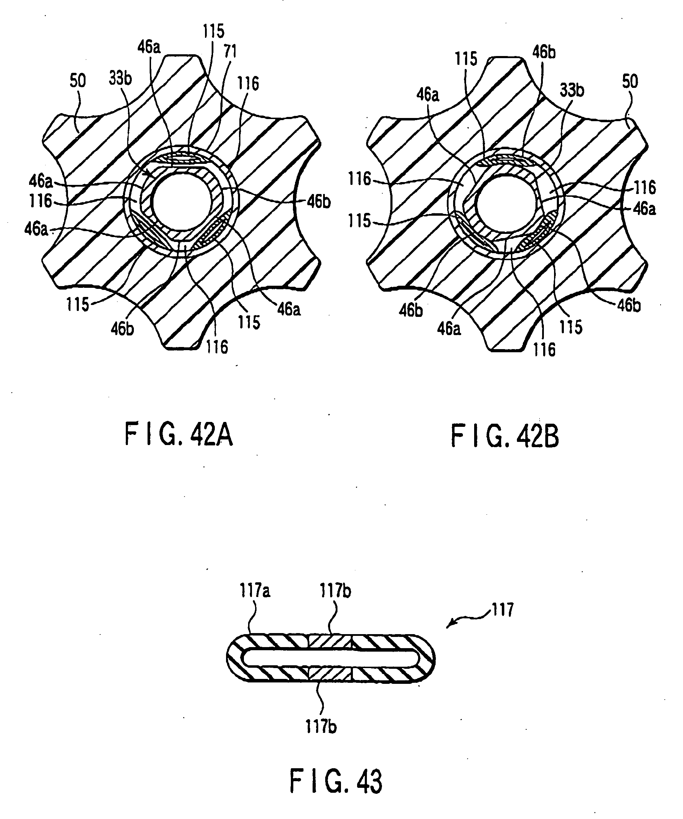

FIGS. 42A and 42B show a first modification of an attachment/detachment portion between the handle unit 4 and the sheath unit 5 of the ultrasonic operating apparatus in the first embodiment. In the first embodiment, the configuration has been shown wherein the conductive rubber ring 94b is provided on the inner peripheral surface of the rotation transmitting member 71 of the handle unit 4. Further, the three corner portions 46b of the outer peripheral flange portion 33b of the sheath unit 5 are brought into contact with the three plane portions 94b1 of the conductive rubber ring 94b, such that the conductive rubber ring 94b is compressed in order to permit conduction between the sheath unit side electric path 40 and the handle unit side electric path 95 via the conductive rubber ring 94b. In the present modification, three metal leaf spring members 115 are provided instead of this the conductive rubber ring 94b. The leaf spring members 115 are provided side by side at equal intervals in a circumferential direction of the inner peripheral surface of the rotation transmitting member 71. Space portions 116 held to stay out of contact with the outer peripheral flange portion 33b of the sheath unit 5 are formed between the leaf spring members 115.

Furthermore, in a situation where the three corner portions 46b of the outer peripheral flange portion 33b correspond to the space portions 116 between the leaf spring members 115 as shown in FIG. 42A, the leaf spring members 115 are held at the non-compression position where they are in the natural state. On the contrary, the handle unit 4 and the sheath unit 5 are relatively rotated in a direction around the central axis of the sheath unit 5, the leaf spring members 115 are switched to the pressure-contact position at which they are placed in pressure-contact by the three corner portions 46b of the outer peripheral flange portion 33b, as shown in FIG. 42B. At this point, the leaf spring members 115 are pressed by the three corner portions 46b of the outer peripheral flange portion 33b, and are thus compressed.

In the present modification, the leaf spring members 115 are held at the non-compression position where they are in the natural state as shown in FIG. 42A during the insertion operation (see FIGS. 31 and 32) in which the outer peripheral flange portion 33b of the sheath unit 5 is inserted straight into the rotation transmitting member 71 when the sheath unit 5 is coupled to the handle unit 4. At this point, the engaging lever 43 on the side of the handle unit 4 is held while being stranded on the inclined surface of the guide groove 41 of the pinch member 32 of the sheath unit 5. Then, the pinch member 32 of the sheath unit 5 is rotated with respect to the handle unit 4 in a direction around the axis, such that the engaging lever 43 on the side of the handle unit 4 engages in an inserted state with the engaging concave portion 42 at one end of the guide groove 41, as shown in FIGS. 33 and 34. At this point, the leaf spring members 115 are pressed by the three corner portions 46b of the outer peripheral flange portion 33b, and are thus compressed, as shown in FIG. 42B. This permits conduction, via the leaf spring members 115, between the sheath unit side electric path 40 and the handle unit side electric path 95. At this point, the second high-frequency electric path 97 for transmitting a high-frequency current is formed in the combination of the sheath unit 5 and the handle unit 4.

Therefore, it is also possible in the present modification to obtain effects similar to those of the attachment/detachment portion between the handle unit 4 and the sheath unit 5 of the ultrasonic operating apparatus in the first embodiment.

FIG. 43 shows a second modification of an attachment/detachment portion between the handle unit 4 and the sheath unit 5 of the ultrasonic operating apparatus in the first embodiment. In the present modification, an urging member 117 shown in FIG. 43 is provided instead of the leaf spring member 115 of the first modification. This urging member 117 has a rubber ring 117a, and a pair of metal contacts 117b. The rubber ring 117a is formed to have a substantially elliptic shape. The pair of metal contacts 117b is separately and oppositely disposed at central positions of the long sides of the ellipse of the rubber ring 117a.

Furthermore, in a situation where the three corner portions 46b of the outer peripheral flange portion 33b correspond to the space portions 116 between the urging members 117, the urging members 117 are held at the non-compression position where they are in the natural state (state in FIG. 43). When the urging members 117 are switched to the pressure-contact position at which they are placed in pressure-contact by the three corner portions 46b of the outer peripheral flange portion 33b, the central portions of the long sides of the ellipse of the rubber rings 117a are pressed by the three corner portions 46b of the outer peripheral flange portion 33b, such that the urging members 117 are compressed. At this point, the pair of metal contacts 117b contacts each other.

Therefore, it is also possible in the present modification to obtain effects similar to those of the attachment/detachment portion between the handle unit 4 and the sheath unit 5 of the ultrasonic operating apparatus in the first embodiment.

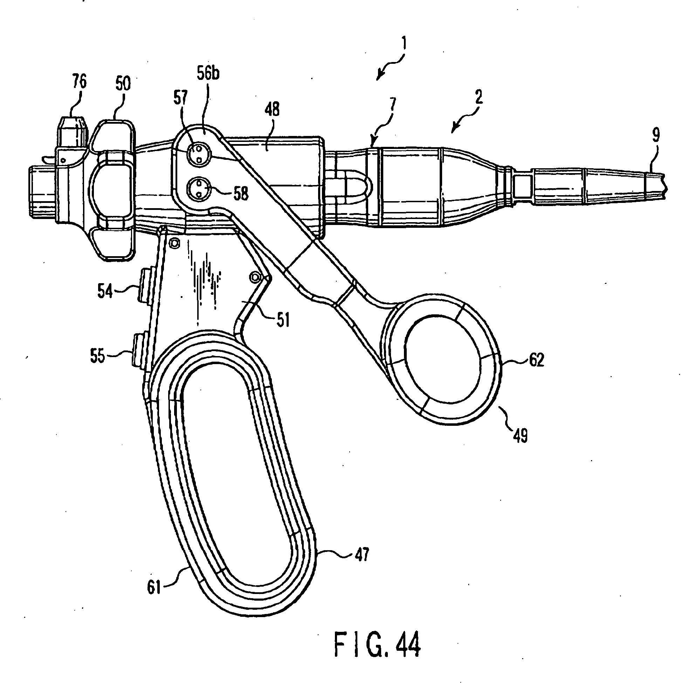

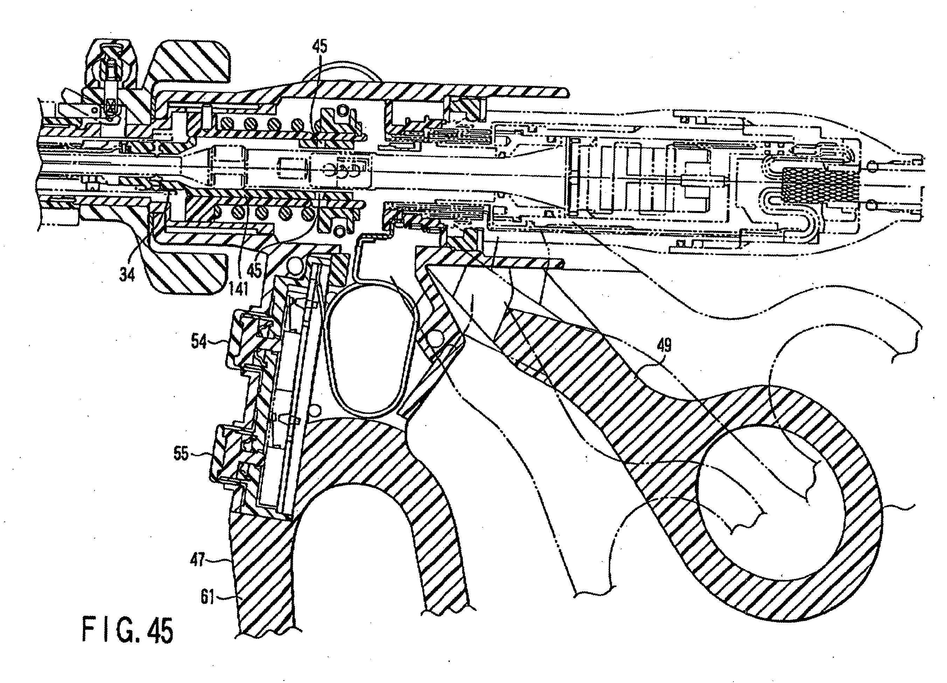

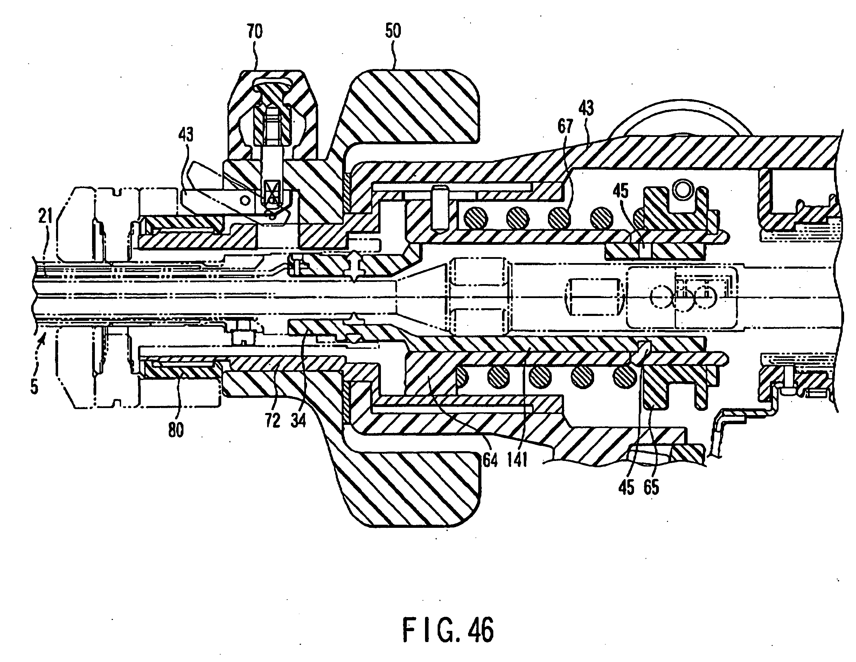

Furthermore, FIGS. 44 to 46 show the configuration of essential parts of the hand piece 1 of an ultrasonic operating apparatus in a second embodiment of the present invention. In the first embodiment (see FIGS. 1 to 41), the configuration has been described wherein the tubular member 98 which permits insulation between the first high-frequency electric path 13 and the second high-frequency electric path 97 when the probe unit 3 is connected to the handle unit 4 is incorporated in the handle unit 4.

In the present embodiment, a tubular member 141 made of an insulating material corresponding to the tubular member 98 in the first embodiment is provided on the side of the sheath unit 5. That is, in the present embodiment, the connecting pipe member 34 of the attachment/detachment mechanism section 31 provided at the proximal end of the sheath main unit 16 of the sheath unit 5 is formed in a coupled state integrally with the tubular member 141 made of the insulating material. At the proximal end of the tubular member 141, there are provided two guide grooves 44 (see FIGS. 12 and 13) with which the engaging pins 45 described later on the side of the handle unit 4 removably engage.

On the inner peripheral surface of the tubular member 141, there are formed three engaging convex portions 99 (see FIG. 21) corresponding to the three engaging concave portions 15 (see FIG. 37) of the flange portion 14 of the probe unit 3. When the probe unit 3 is connected to the handle unit 4, the three engaging convex portions 99 of the tubular member 141 of the sheath unit 5 removably engage with the three engaging concave portions 15 of the flange portion 14 of the probe unit 3. This regulates the positions of the probe unit 3 and the tubular member 141 of the sheath unit 5 in the rotation direction. Thus, the combination of the probe unit 3 and the transducer unit 2 is driven to integrally rotate together with the set unit inside the holding cylinder 48 during the rotational operation of the swing operation knob 50.

In addition, the configuration is substantially the same as that in the first embodiment except for this modified part. Therefore, in FIGS. 44 to 46, the same signs are assigned to the same parts as those in the first embodiment, and such parts will not be described.

Thus, in the present embodiment, the tubular member 141 is provided on the side of the sheath unit 5. Therefore, the tubular member 141 can be replaced together with the inexpensive sheath unit 5 in the case where the part engaging with the probe unit 3 in the tubular member 141 is damaged when the combination of the probe unit 3 and the transducer unit 2 is driven to integrally rotate together with the set unit inside the holding cylinder 48 during the rotational operation of the swing operation knob 50. As a result, when the tubular member 141 is damaged, the hand piece 1 can be repaired at low costs because it is not necessary to replace the expensive handle unit 4.

Furthermore, the tubular member 141 is provided on the side of the sheath unit 5, such that the connecting pipe member 34 of the sheath unit 5 can be formed integrally with the tubular member 141 made of the insulating material. Therefore, the number of components within the handle unit 4 can be lower than in the first embodiment, so that the internal configuration of the holding cylinder 48 of the handle unit 4 can be simplified. As a result, the axial length of the entire holding cylinder 48 of the handle unit 4 can be smaller than in the first embodiment, thereby making it possible to reduce the size of the entire hand piece 1.

It is to be noted that the present invention is not limited to the embodiments described above, and needless to say, various modifications can be made without departing from the scope of the present invention as defined in the claims.

## Claims

1. An ultrasonic operating apparatus **characterized by** comprising:
an ultrasonic transducer (6) which generates ultrasonic vibrations;
a probe portion (3) which has a distal end and a proximal end, the proximal end being coupled to the ultrasonic transducer (6), ultrasonic waves output from the ultrasonic transducer (6) being transmitted to the probe portion (3);
a sheath portion (5) having a distal end and a proximal end and into which the probe portion (3) is removably inserted, the sheath portion (5) having a jaw (17) swingably supported on the distal end thereof to be opposite to the probe portion (3);
a handle portion (4) which is detachably coupled to the proximal end of the sheath portion (5) and which opens/closes the jaw (17) with respect to the probe portion (3);
a first high-frequency electric path (97) having a sheath portion side electric path (40) disposed on the side of the sheath portion (5) and a handle portion side electric path (95) disposed on the side of the handle portion (4);
an outer peripheral flange portion (33b) which is disposed at the proximal end of the sheath portion (5) and which is formed on the outer periphery of the sheath portion (5) and which is connected to the sheath portion side electric path (40); and
an engaging portion which is disposed inside the handle portion (4) and which removably engages with the outer peripheral flange portion (33b),
the engaging portion comprising:
an insertion hole forming portion into which the outer peripheral flange portion (33b) is inserted when the sheath portion (5) is coupled to the handle portion (4); and
an urging portion, which is conductive and disposed in the insertion hole forming portion, the urging portion being brought into pressure-contact with the flange portion (33b) when the flange portion (33b) and the engaging portion are connected.

2. The ultrasonic operating apparatus according to claim 1, **characterized in that**
the outer peripheral flange portion (33b) has an outer peripheral portion formed into a non-circular irregular shape; and
the urging portion is disposed inside the handle portion (4), the urging portion being held at a non-pressure-contact position with respect to the outer peripheral flange portion (33b) at an insertion operation position at which the outer peripheral flange portion (33b) is inserted into the insertion hole forming portion along the axial direction of the sheath portion (5), the urging portion being switched to a pressure-contact position along with the rotational operation of the outer peripheral flange portion (33b) from the insertion operation position in a direction around the central axis of the sheath portion (5).

3. The ultrasonic operating apparatus according to claim 2, **characterized in that**
the urging portion has a ring-shaped rubber portion (94b); and
the rubber portion (94b) is brought into pressure-contact with the outer
peripheral flange portion (33b) to urge the outer peripheral flange portion (33b) in a direction to permit conduction between the sheath portion side electric path (40) and the handle portion side electric path (95) when the sheath portion (5) is connected to the handle portion (4).

4. The ultrasonic operating apparatus according to claim 3, **characterized in that**
the rubber portion (94b) is formed by conductive rubber.

5. The ultrasonic operating apparatus according to claim 1, **characterized in that**
the urging portion has a leaf-spring-shaped urging member (115); and
the urging member (115) is brought into pressure-contact with the outer
peripheral flange portion (33b) to urge the outer peripheral flange portion (33b) in a direction to permit conduction between the sheath portion side electric path (40) and the handle portion side electric path (95) when the sheath portion (5) is connected to the handle portion (4).

6. An ultrasonic operating apparatus according to claim 1, **characterized by** further comprising:
a second high-frequency electric path (13) which is provided in a combination of the ultrasonic transducer (6) and the probe portion (3) and which transmits a high-frequency current.

7. The ultrasonic operating apparatus according to claim 6, **characterized in that**
the handle portion (4) has a rotational operation portion (50) which rotationally drives the sheath portion (5) in a direction around the central line of the sheath portion (5); and
the transducer connecting portion has a tubular member (98) formed by an insulating material which permits insulation between the probe portion (3) and the first high-frequency electric path (97) when the probe portion (3) is connected to the handle portion (4).

8. The ultrasonic operating apparatus according to claim 7, **characterized in that**
the tubular member (98) has a position regulating mechanism which regulates the rotational position of the probe when the probe portion (3) is connected to the handle portion (4).

9. The ultrasonic operating apparatus according to claim 8, **characterized in that**
the position regulating mechanism has a protrusion (99) provided to inwardly protrude on the inner peripheral surface of the tubular member (98); and
an engaging concave portion (15) which is formed in the outer peripheral surface of the probe portion (3) and which engages with the protrusion (99) of the tubular member (98).

10. The ultrasonic operating apparatus according to claim 6, **characterized in that**
the probe portion (3) has, at the distal end thereof, a distal end curved portion which is curved in a direction deviating from the direction of the central line of the probe portion (3); and
the jaw (17) has a distal end curved portion which is curved to have a shape corresponding to the distal end curved portion of the probe portion (3).

11. The ultrasonic operating apparatus according to claim 6, **characterized in that**
the handle portion (4) has a rotational operation portion (50) which rotationally drives the sheath portion (5) in a direction around the central line of the sheath portion (5); and
the sheath portion (5) has a tubular member (98) formed by an insulating material which permits insulation between the probe portion (3) and the first high-frequency electric path (97) when the probe portion (3) is connected to the handle portion (4).

## Patentansprüche

1. Ultraschallbetriebsvorrichtung, **gekennzeichnet durch**
einen Ultraschallwandler (6), der Ultraschallvibrationen erzeugt;
ein Geberteil (3), das ein Distalende und ein Proximalende aufweist, wobei das Proximalende mit dem Ultraschallwandler (6) gekoppelt ist und von dem Ultraschallwandler (6) ausgegebene Ultraschallwellen zu dem Geberteil (3) übertragen werden;
ein Hüllenteil (5), das ein Distalende und ein Proximalende aufweist und in welches das Geberteil (3) lösbar eingefügt ist, wobei das Hüllenteil (5) an seinem Distalende eine schwingbar gelagerte Klemme (17) aufweist, die gegenüber dem Geberteil (3) liegt;
ein Handhabungsteil (4), das lösbar mit dem Proximalende des Hüllenteils (5) gekoppelt ist und das die Klemme (17) hinsichtlich des Geberteils (3) öffnet/schließt;
einen ersten elektrischen Hochfrequenzpfad (97), der einen an der Seite des Hüllenteils (5) verlaufenden elektrischen hüllenteilseitigen Pfad (40) und einen an der Seite des Handhabungsteils (4) verlaufenden elektrischen handhabungsteilseitigen Pfad (95) aufweist;
ein äußeres Flanschteil (33b), das an dem Proximalende des Hüllenteils (5) angeordnet, außen an dem Hüllenteil (5) gebildet und mit dem elektrischen hüllenteilseitigen Pfad (40) verbunden ist; und
ein Eingriffsteil, das innerhalb des Handhabungsteils (4) angeordnet ist und das lösbar mit dem äußeren Flanschteil (33b) in Eingriff tritt,
wobei das Eingriffsteil aufweist:
ein Einfügungslochbildungsteil, in welches das äußere Flanschteil (33b) eingefügt wird, wenn das Hüllenteil (5) mit dem Handhabungsteil (4) gekoppelt ist; und
ein Druckteil, das leitfähig ist und in dem Einfügungslochbildungsteil angeordnet ist, wobei das Druckteil mit dem Flanschteil (33b) in Druckkontakt gebracht wird, wenn das Flanschteil (33b) und das Eingriffsteil verbunden sind.

2. Ultraschallbetätigungsvorrichtung nach Anspruch 1,
**dadurch gekennzeichnet, dass**
das äußere Flanschteil (33b) ein äußeres Teil mit einer nicht-kreisförmigen, irregulären Form aufweist; und
das Druckteil in dem Handhabungsteil (4) angeordnet ist, wobei das Druckteil bei einer Einfügungsbetriebsposition, bei der das äußere Flanschteil (33b) entlang der Axialrichtung des Hüllenteils (5) in das Einfügungslochbildungsteil eingefügt wird, hinsichtlich des äußeren Flanschteils (33b) in einer Nichtdruckkontaktposition gehalten wird, wobei das Druckteil aus der Einfügungsbetriebsposition mit der Drehbetätigung des äußeren Flanschteils (33b) in eine Richtung um die Mittelachse des Hüllenteils (5) in eine Druckkontaktposition verschoben wird.

3. Ultraschallbetätigungsvorrichtung nach Anspruch 2,
**dadurch gekennzeichnet, dass**
das Druckteil ein ringförmiges Gummiteil (94b) aufweist; und
das Gummiteil (94b) zum Drücken des äußeren Flanschteils (33b) in eine Richtung, bei der eine Leitung zwischen dem elektrischen hüllenteilseitigen Pfad (40) und dem elektrischen handhabungsteilseitigen Pfad (95), wenn das Hüllenteil (5) mit dem Handhabungsteil (4) verbunden ist, ermöglicht wird, in Druckkontakt mit dem äußeren Flanschteil (33b) gebracht wird.

4. Ultraschallbetätigungsvorrichtung nach Anspruch 3,
**dadurch gekennzeichnet, dass**
das Gummiteil (94b) aus leitfähigem Gummi gebildet ist.

5. Ultraschallbetätigungsvorrichtung nach Anspruch 1,
**dadurch gekennzeichnet, dass**
das Druckteil ein blattfederförmiges Druckelement (115) aufweist; und
das Druckelement (115) zum Drücken des äußeren Flanschteils (33b) in eine Richtung, bei der eine Leitung zwischen dem elektrischen hüllenteilseitigen Pfad (40) und dem elektrischen handhabungsteilseitigen Pfad (95), wenn das Hüllenteil (5) mit dem Handhabungsteil (4) verbunden ist, ermöglicht wird, in Druckkontakt mit dem äußeren Flanschteil (33b) gebracht wird.

6. Ultraschallbetätigungsvorrichtung nach Anspruch 1,
ferner **gekennzeichnet durch**
einen zweiten elektrischen Hochfrequenzpfad (13), der in Kombination des Ultraschallwandlers (6) und des Geberteils (3) bereitgestellt ist und einen Hochfrequenzstrom leitet.

7. Ultraschallbetätigungsvorrichtung nach Anspruch 6,
**dadurch gekennzeichnet, dass**
das Handhabungsteil (4) ein Drehbetätigungsteil (50) aufweist, welches das Hüllenteil (5) in eine Richtung um die Mittellinie des Hüllenteils (5) dreht; wobei
das Wandlerverbindungsteil ein Röhrenelement (98) aufweist, welches aus einem isolierenden Material gebildet ist und Isolation zwischen dem Geberteil (3) und dem elektrischen Hochfrequenzpfad (7) ermöglicht, wenn das Geberteil (3) mit dem Handhabungsteil (4) verbunden ist.

8. Ultraschallbetätigungsvorrichtung nach Anspruch 7,
**dadurch gekennzeichnet, dass**
das Röhrenelement (98) einen Positionssteuerungsmechanismus aufweist, der die Drehposition des Gebers steuert, wenn das Geberteil (3) mit dem Handhabungsteil (4) verbunden ist.

9. Ultraschallbetätigungsvorrichtung nach Anspruch 8,
**dadurch gekennzeichnet, dass**
der Positionssteuerungsmechanismus einen Vorsprung (99) aufweist, der von der inneren Oberfläche des Röhrenelements (98) nach Innen hervorsteht; und durch
ein konkaves Eingriffsteil (15), das an der äußeren Oberfläche des Geberteils (3) gebildet ist und mit dem Vorsprung (99) des Röhrenelements (98) in Eingriff tritt.

10. Ultraschallbetätigungsvorrichtung nach Anspruch 6,
**dadurch gekennzeichnet, dass**
das Geberteil (3) an seinem Distalende ein kurvenförmiges Distalendteil aufweist, welches in einer von der Richtung der Mittellinie des Geberteils (3) abweichenden Richtung gebogen ist; wobei
die Klemme (17) ein kurvenförmiges Distalendteil aufweist, das gebogen ist und eine dem kurvenförmigen Distalendteil des Geberteils (3) entsprechende Form aufweist.

11. Ultraschallbetätigungsvorrichtung nach Anspruch 6,
**dadurch gekennzeichnet, dass**
das Handhabungsteil (4) ein Drehbetätigungsteil (50) aufweist, welches das Hüllenteil (5) in eine Richtung um die Mittellinie des Geberteils (5) dreht; und
das Hüllenteil (5) ein Röhrenelement (98) aufweist, welches aus einem isolierenden Material gebildet ist und Isolation zwischen dem Geberteil (3) und dem ersten elektrischen Hochfrequenzpfad (97) ermöglicht, wenn das Geberteil (3) mit dem Handhabungsteil (4) verbunden ist.

## Revendications

1. Appareil d'intervention à ultrasons, **caractérisé par le fait qu'**il comprend :
un transducteur à ultrasons (6) qui génère des vibrations ultrasonores ;
une partie de sonde (3) qui comporte une extrémité distale et une extrémité proximale, l'extrémité proximale étant couplée au transducteur à ultrasons (6), des ondes ultrasonores délivrées par le transducteur à ultrasons (6) étant transmises à la partie de sonde (3) ;
une partie de gaine (5) comportant une extrémité distale et une extrémité proximale, et dans laquelle la partie de sonde (3) est introduite de manière amovible, la partie de gaine (5) comportant une mâchoire (17) supportée de manière pivotante sur son extrémité distale pour se trouver opposée à la partie de sonde (3) ;
une partie de poignée (4) qui est couplée de manière amovible à l'extrémité proximale de la partie de gaine (5) et qui ouvre/ferme la mâchoire (17) par rapport à la partie de sonde (3) ;
un premier trajet électrique à haute fréquence (97) comportant un trajet électrique de côté partie de gaine (40) disposé sur le côté de la partie de gaine (5) et un trajet électrique de côté partie de poignée (95) disposé sur le côté de la partie de poignée (4) ;
une partie de rebord périphérique extérieur (33b) qui est disposée au niveau de l'extrémité proximale de la partie de gaine (5) et qui est formée sur la périphérie extérieure de la partie de gaine (5) et qui est connectée au trajet électrique de côté partie de gaine (40) ; et
une partie d'engagement qui est disposée à l'intérieur de la partie de poignée (4) et qui engage de manière amovible la partie de rebord périphérique extérieur (33b),
la partie d'engagement comprenant :
une partie de formation de trou d'introduction dans laquelle la partie de rebord périphérique extérieur (33b) est introduite lorsque la partie de gaine (5) est couplée à la partie de poignée (4) ; et
une partie de poussée, qui est conductrice et qui est disposée dans la partie de formation de trou d'introduction, la partie de poussée étant amenée en contact par pression avec la partie de rebord (33b) lorsque la partie de rebord (33b) et la partie d'engagement sont connectées.

2. Appareil d'intervention à ultrasons selon la revendication 1, **caractérisé en ce que**
la partie de rebord périphérique extérieur (33b) comporte une partie périphérique extérieure réalisée selon une forme irrégulière non circulaire ; et
la partie de poussée est disposée à l'intérieur de la partie de poignée (4), la partie de poussée étant maintenue dans une position de contact sans pression par rapport à la partie de rebord périphérique extérieur (33b) dans une position d'opération d'introduction dans laquelle la partie de rebord périphérique extérieur (33b) est introduite dans la partie de formation de trou d'introduction dans la direction axiale de la partie de gaine (5), la partie de poussée passant dans une position de contact par pression lors de l'opération de rotation de la partie de rebord périphérique extérieur (33b) à partir de la position d'opération d'introduction dans une direction autour de l'axe central de la partie de gaine (5).

3. Appareil d'intervention à ultrasons selon la revendication 2, **caractérisé en ce que**
la partie de poussée comporte une partie en caoutchouc en forme d'anneau (94b) ; et
la partie en caoutchouc (94b) est amenée en contact par pression avec la partie de rebord périphérique extérieur (33b) pour pousser la partie de rebord périphérique extérieur (33b) dans une direction visant à permettre une conduction entre le trajet électrique de côté partie de gaine (40) et le trajet électrique de côté partie de poignée (95) lorsque la partie de gaine (5) est connectée à la partie de poignée (4).

4. Appareil d'intervention à ultrasons selon la revendication 3, **caractérisé en ce que** la partie en caoutchouc (94b) est formée par un caoutchouc conducteur.

5. Appareil d'intervention à ultrasons selon la revendication 1, **caractérisé en ce que**
la partie de poussée comporte un élément de poussée en forme de ressort à lame (115) ; et
l'élément de poussée (115) est amené en contact par pression avec la partie de rebord périphérique extérieur (33b) pour pousser la partie de rebord périphérique extérieur (33b) dans une direction visant à permettre une conduction entre le trajet électrique de côté partie de gaine (40) et le trajet électrique de côté partie de poignée (95) lorsque la partie de gaine (5) est connectée à la partie de poignée (4).

6. Appareil d'intervention à ultrasons selon la revendication 1, **caractérisé par le fait qu'**il comprend en outre :
un second trajet électrique à haute fréquence (13) qui est obtenu par une combinaison du transducteur à ultrasons (6) et de la partie de sonde (3) et qui transmet un courant à haute fréquence.

7. Appareil d'intervention à ultrasons selon la revendication 6, **caractérisé en ce que**
la partie de poignée (4) comporte une partie d'opération de rotation (50) qui entraîne en rotation la partie de gaine (5) dans une direction autour de l'axe central de la partie de gaine (5) ; et
la partie de connexion de transducteur comporte un élément tubulaire (98) formé par un matériau isolant qui permet une isolation entre la partie de sonde (3) et le premier trajet électrique à haute fréquence (97) lorsque la partie de sonde (3) est connectée à la partie de poignée (4) .

8. Appareil d'intervention à ultrasons selon la revendication 7, **caractérisé en ce que**
l'élément tubulaire (98) comporte un mécanisme de réglage de position qui règle la position en rotation de la sonde lorsque la partie de sonde (3) est connectée à la partie de poignée (4).

9. Appareil d'intervention à ultrasons selon la revendication 8, **caractérisé en ce que**
le mécanisme de réglage de position comporte une saillie (99) réalisée pour faire saillie vers l'intérieur sur la surface périphérique intérieure de l'élément tubulaire (98) ; et
une partie concave d'engagement (15) est formée dans la surface périphérique extérieure de la partie de sonde (3) et engage avec la saillie (99) de l'élément tubulaire (98).

10. Appareil d'intervention à ultrasons selon la revendication 6, **caractérisé en ce que**
la partie de sonde (3) comporte, au niveau de son extrémité distale, une partie courbée d'extrémité distale qui est courbée dans une direction qui va en s'écartant de la direction de l'axe central de la partie de sonde (3) ; et
la mâchoire (17) comporte une partie courbée d'extrémité distale qui est courbée pour avoir une forme qui correspond à la partie courbée d'extrémité distale de la partie de sonde (3).

11. Appareil d'intervention à ultrasons selon la revendication 6, **caractérisé en ce que**
la partie de poignée (4) comporte une partie d'opération de rotation (50) qui entraîne en rotation la partie de gaine (5) dans une direction autour de l'axe central de la partie de gaine (5) ; et
la partie de gaine (5) comporte un élément tubulaire (98) formé par un matériau isolant qui permet une isolation entre la partie de sonde (3) et le premier trajet électrique à haute fréquence (97) lorsque la partie de sonde (3) est connectée à la partie de poignée (4).
